# EUROPEAN PATENT SPECIFICATION

(11) **EP 2 497 831 B1**
(45) Date of publication and mention of the grant of the patent: **16.07.2014**
(21) Application number: 11008462.1
(22) Date of filing: 25.05.2005
(51) Int. Cl.: C12N 15/63, A61K 39/00, A61K 39/04, A61K 39/21, C12N 15/86, C07K 16/08, C12N 7/00, C07K 14/005

(54) **TB vaccination using HCMV-based vaccine vectors**
TB-Impfung unter Verwendung von Impfvektoren auf HCMV-Basis
Vaccin contre la Tuberculosis utilisant des vecteurs de vaccin à base de HCMV

(30) Priority: 25.05.2004 US 574493 P
(43) Date of publication of application: 12.09.2012
(62) Divisional of application: 05758631.5
(73) Proprietor: Oregon Health and Science University, Portland, Oregon 97201 (US)
(72) Inventor: Picker, Louis J., Portland, OR 97201 (US); Jarvis, Michael, Portland, OR 97201 (US); Nelson, Jay A., Portland, OR 97201 (US); Hansen, Scott G., Portland, OR 97210 (US)
(74) Representative: Schüssler, Andrea

(56) References cited:
- WO-A2-02/062296
- ULMER B J: "TUBERCULOSIS DNA VACCINES", SCANDINAVIAN JOURNAL OF INFECTIOUS DISEASES, SCANDINAVIAN UNIVERSITY PRESS, OSLO, NO, vol. 38, 1 January 2001 (2001-01-01), pages 246-248, XP008078398, ISSN: 0036-5548, DOI: 10.1080/003655401300077162
- WANG X ET AL: "Murine cytomegalovirus abortively infects human dendritic cells, leading to expression and presentation of virally vectored genes", JOURNAL OF VIROLOGY, THE AMERICAN SOCIETY FOR MICROBIOLOGY, US, vol. 77, no. 13, 1 July 2003 (2003-07-01), pages 7182-7192, XP003008533, ISSN: 0022-538X, DOI: 10.1128/JVI.77.13.7182-7192.2003
- RIZAVANOV A A ET AL: "Generation of a recombinant cytomegalovirus for expression of a hantavirus glycoprotein", JOURNAL OF VIROLOGY, THE AMERICAN SOCIETY FOR MICROBIOLOGY, US, vol. 77, no. 22, 1 November 2003 (2003-11-01), pages 12203-12210, XP003008534, ISSN: 0022-538X, DOI: 10.1128/JVI.77.22.12203-12210.2003

## Description

### FIELD OF THE INVENTION

Aspects of the present invention relate generally to protection against TB and more particularly to novel vaccine vectors, based on HCMV, for protection against TB.

### BACKGROUND

*The HIV epidemic.* The human immunodeficiency virus (HIV) epidemic likely comprises the single most serious threat to public health in modern times (arguably in recorded history). The worldwide toll from this virus will likely exceed 68 million by 2020, with high incidence of disease in third world countries undermining the social fabric. Anti-retroviral therapy, while having a tremendous impact on the course of disease in aggressively treated individuals, is expensive, difficult to implement on a large scale, and most importantly, not curative. Therefore, it is generally conceded that control of the HIV epidemic will not be possible until the development of an effective prophylactic vaccine. Unfortunately, the biology of the virus is problematic. In natural human HIV infection, or experimental infection of non-human primates (NHP) with the closely related simian immunodeficiency virus (SIV), infection is almost always progressive despite both cellular and humoral immune responses, and situations consistent with immunologic control of infection are uncommon and transient. In the NHP models, relatively strong vaccine-elicited cellular immune responses have shown promise in the control of certain pathogenic strains, but the longevity of the protective responses, the durability of the protection after exposure, and the general applicability of these results to the highly diverse, CCR5-tropic HIV strains likely to be encountered in the "real world" is uncertain. The rapid replication of these viruses and their profound genetic flexibility allow unrivaled evolution and adaptation to selection pressure. This capacity, together with the ability of these viruses to establish latency, results in a formidable barrier to immune control. It is therefore increasingly evident that if such control is to be established, it will take a quantitatively and qualitatively optimized immune response, likely including both cellular and humoral immunity. Such optimized immunity will necessarily be maintained for decades, able to immediately suppress and control any viral challenge before the virus can manifest escape.

From the humoral immune system perspective, it is increasingly apparent that high titre, neutralizing Abs (nAbs) can provide significant anti-viral activity, although the current ability to generate such responses with vaccines remains highly problematic. With regard to cellular immunity, the specific nature of such optimized responses remain speculative. However, based on clinical observations of HIV infection and data obtained from a variety of animal models of chronic viral infection (including SIV/SHIV models in NHP), it is anticipated that effective T cell responses will necessarily be 1) large in size (*e*.*g*., high frequency), 2) epitopically broad, 3) appropriately distributed (for example, "pre-positioned" at potential sites of viral entry and initial replication), 4) sensitive to low epitope densities (*e*.*g*., possess high functional avidity), 5) functionally appropriate (*e*.*g*., with regard to cytokine synthesis, cytotoxicity) and 6) maintained as such (e.g., 1-5) indefinitely. The latter point is especially important, as the rapid replication and evolution capabilities of these lentiviruses are such that even the rapid effector cell precursor expansions associated with a memory response may come too late to prevent immune escape and viral outgrowth.

In recent years the field has focused on the induction of strong anti-viral cellular immunity using *replication incompetent* viral vectors *(e.g.,* vaccinia derivatives, adenovirus), either alone or following priming with Ag-encoding DNA. Despite the relative inability of these approaches to generate high titre nAbs, such vaccines have been effective in providing significant cell-mediated protection against certain challenges (see below). They have not, however, been particularly effective in controlling chronic-aggressive SIVs (*e*.*g*., SIVmac239) in NHPs, and the longevity of protective responses pre-challenge and durability of protection after challenge has not been rigorously explored. Indeed, at this point, the best "protection" has not been generated by these approaches, but rather by vaccination of NHP with *attenuated* SIVs *(e.g.,* Δnef SIV), which have protected against aggressive SIV challenge. Unfortunately, for the live attenuated SIV approach to work, the attenuation needs to be modest, and the potential for full pathogenicity and disease by the vaccine itself has proved unacceptably high. If the protection afforded by attenuated SIV exposure is indeed immunologically mediated (and increasing evidence suggests it is—see herein below), the relative effectiveness of this approach suggests that chronic/continuous vector infection might have quantitative or qualitative advantages over the more episodic/limited Ag exposure afforded by conventional prime-boost strategies.

Therefore, there is a pronounced need in the art for SIV and HIV vaccination vectors capable of chronic infection, high immunogenicity, and yet unable or highly unlikely to cause significant disease by themselves.

Wang et al., Journal of Virology, Vol. 77, No. 13, July 2003, pp. 7182-7192 describe that murine cytomegalovirus abortively infects human dendritic cells, leading to expression and presentation of virally vectored genes.

Rizvanov et al., Journal of Virology, Vol. 77, No. 22, Nov. 2003, pp. 12203-12210 describe the generation of a recombinant cytomegalovirus for the expression of a hantavirus glycoprotein.

US 2002/0176870 A1 concerns methods and compositions useful for stimulating an immune response.

### SUMMARY OF THE INVENTION

Particular aspects provide for use of the β-herpesvirus Cytomegalovirus (CMV: HCMV) as a uniquely evolved "vector" for safely initiating and indefinitely maintaining high level cellular and humoral immune responses (against, *e.g.,* TB).

CMV (*e*.*g*., HCMV) is engineerable to express SIV or HIV determinants in highly immunogenic form, and to invoke high level, persistent cellular and humoral immunity in vaccinated subjects, even those that have had prior CMV exposure.

The immunity to SIV and HIV determinants is superior in intensity, persistence, and/or "quality" (*e.g.,* functional attributes and localization) to prior art approaches, resulting in more effective control of pathogenic SIV and HIV challenges

According to further aspects, repeated vaccinations with the same core CMV-based vector containing different/modified Ags is feasible and productive, because of CMV's highly evolved capability to "slip" by the immune response, thereby allowing either the *de novo* generation of unrelated responses, or responses to new epitopes contained within a modified Ag,

According to yet further aspects, the wildtype CMV genome is modifiable to enhance safety without sacrificing immunogenicity and persistence.

Particular aspects of the disclosure provide a method for treatment or prevention of HIV, SIV or TB, comprising infection of a subject in need thereof with at least one recombinant CMV-based vector (e.g., HCMV or RhCMV) comprising an expressible HIV/SIV/TB antigen or a variant or fusion protein thereof. In particular embodiments of the method, infection is of an immunocompetent, HCMV or RhCMV seropositive subject.

In certain embodiments, the at least one TB antigen is selected from the group consisting of ESAT-6, Ag85A, AG85B, MPT51, MPT64, CFP10, TB10.4, Mtb8.4, hspX, CFP6, Mtb12, Mtb9.9 antigens, Mtb32A, PstS-1, PstS-2, PstS-3, MPT63, Mtb39, Mtb41, MPT83, 71-kDa, PPE 68, LppX, and antigenic portions, variants and fusion proteins thereof.

Additional embodiments comprise serial re-infection with at least one recombinant HCMV vector comprising an expressible TB antigen or a variant or fusion protein thereof. In certain embodiments, the expressible TB antigen, or variant or fusion protein thereof, of the serial re-infection vector is different than that of the initial infection vector. In some embodiments, expression is driven by an antigen encoding sequence in operable association with a promoter selected from the group consisting of a constitutive CMV promoter, an immediate early CMV promoter, an early CMV promoter and a late CMV promoter. In particular embodiments, the promoter is selected from the group consisting of EF1-alpha, MIE, pp65 and gH.

Further aspects provide a recombinant HCMV vaccine vector, comprising an expressible TB antigen or a variant or fusion protein thereof. Preferably, the vector comprises 'suicide' or 'safety' means. In particular vector embodiments, expression is driven by an antigen encoding sequence in operable association with a promoter selected from the group consisting of a constitutive CMV promoter, an immediate early CMV promoter, an early CMV promoter and a late CMV promoter. In some embodiments, the promoter is selected from the group consisting of EF1-alpha, MIE, pp65 and gH.

Additionally provided are pharmaceutical compositions, comprising, along with a pharmaceutically acceptable carrier or excipient, a recombinant HCMV comprising an expressible TB antigen or a variant or fusion protein thereof. Preferably, the recombinant HCMV vaccine vector comprises suicide or safety means.

### BRIEF DESCRIPTION OF THE DRAWINGS

Figures 1A and 1B show, according to particular aspects, a summary of applicants' analysis of T cell immunity to HCMV.
Figures 2A, 2B and 2C show, according to particular aspects, robust peripheral blood T cell responses to RhCMV (2A), peripheral blood T cell responses to RhCMV in 27 adult males (2B), and that RhCMV-specific memory T cells are highly enriched in spleen and lung (2C).
Figure 3 shows, according to particular aspects, immunologic evidence and proof of RhCMV re-infection, and depicts CMV-specific CD4+ and CD8+ T cell frequencies/proliferative status in blood and plasma Ab titers after "re-infection."
Figures 4A and 4B show, according to particular aspects, a schematic of Cre/LoxP-based recombination for construction of RhCMVvLoxP-based RhCMV/SIVmac239gag recombinants.
Figure 5 shows, according to particular aspects, RhCMV/SIVgag re-infection of RhCMV-seropositive RMs.
Figures 6A, 6B, 6C and 6D show, according to particular aspects, induction and boosting of gag T cell and Ab responses by RhCMV-vectored SIVgag in the setting of re-infection.
Figure 7 shows, according to particular aspects, a comparison of gag-specific T cell frequencies in SIV(Δnef) and RhCMV(gag)-immunized RM. In both groups, both Cd4+ and Cd8+ gag-specific T cell responses were higher in lung as compared to blood, even through the percentages in blood were memory corrected.

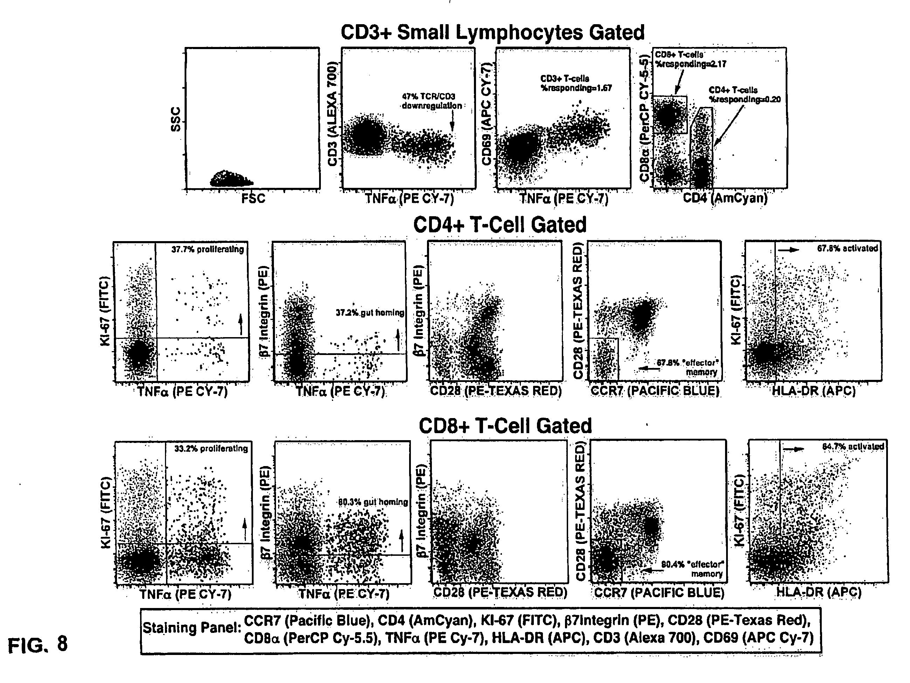

Figure 8 shows, according to particular aspects, quantification and characterization of a young adult RM peripheral blood CD4+ and CD8+ T cell responses to RhCMV IE-1 15mer mix by "1O color" cytokine flow cytometry.
Figure 9 shows, according to particular aspects, construction of RhCMV and HCMV vaccine vectors. Heterologous pathogen antigen(s) are inserted into RhCMV or HCMV bacterial artificial chromosomes (BACs) by E/T and Flp-mediated recombination.
Figure 10 shows, according to particular aspects, an exemplary tetracycline-regulated RhCMV/HCMV 'safety' vaccine vector. This RhCMV/ HCMV safety vector contains two interactive genetic components within the RhCMV/HCMV genome that together enable Tet-induced vector inactivation.
Figure 11 shows, according to particular aspects, another exemplary tetracycline-regulated RhCMV/HCMV 'safety' vaccine vector. Such RhCMV/HCMV vaccine vectors are constructed by placing a gene essential for virus replication, (in this example, *Rh70* (HCMV homologue-*UL44*); DNA polymerase processivity factor), under control of the Tet-inducible system described in Figure 10.
Figure 12 shows, according to particular aspects, another exemplary tetracycline-regulated RhCMV/HCMV 'safety' vaccine vector. Such RhCMV/HCMV vaccine vectors are constructed containing a cytotoxic gene (CytoG) under control of the Tet-inducible system as detailed in Figure 11. After inoculation of animals with Tet-regulated vectors in the absence of Dox, virus replication can be rapidly inactivated by Dox-mediated induction of the cytopathic gene resulting in death of the vaccine vector-infected cell.
Figure 13 shows, according to particular aspects, construction of exemplary RhCMV and HCMV gene therapy vectors. Therapeutic gene(s) are inserted into RhCMV or HCMV bacterial artificial chromosomes (BACs) by E/T and Flp-mediated recombination. The schematic shows a generalized strategy for insertion of an epitope-tagged therapeutic gene into the non-coding region between rh213 and Rh214 of RhCMV. This strategy can be similarly used for insertion of therapeutic genes at other defined sites within the RhCMV/HCMV genome

### DETAILED DESCRIPTION OF THE INVENTION

Particular aspects provide for use of recombinant β-herpesvirus Cytomegaloviruses (CMV) as a uniquely evolved "vector" for safely initiating and indefinitely maintaining high level cellular and humoral immune responses against currently intractable conditions (*e.g.*, tuberculosis, etc.).

According to particular aspects of the present invention, such novel use of CMV vectors (*e.g.*, HCMV) is premised upon the following factors:
(1) CMV elicits an astoundingly high frequency (steady-state) T cell response, at least an order of magnitude higher than that of any non-persistent virus (it is not uncommon for CMV-specific T cells to encompass >20% of the circulating memory repertoire), and the representation of CMV-specific T cells is even higher in tissues such as the lung and liver;
(2) CMV elicits high-titre nAb (neutralizing antibody) responses, including at mucosal surfaces;
(3) The above responses are maintained indefinitely;
(4) CMV is capable of re-infecting already chronically infected individuals, even in the face of high level immunity, and such re-infection not only potently and permanently boosts existing CMV-specific responses (likely accounting for the high frequencies of these responses in long-term infected adults), but is also capable of rapidly inducing new responses to distinct CMV-encoded epitopes/Ags (indeed, according to particular aspects, serial re-infections are possible, so that new responses to a series of distinct Ags or epitopes can be repeatedly generated using the same core vector);
(5) CMV engenders pathogenicity only in very specific (and overall, quite rare) situations of immune deficiency or immaturity (its potential for disease is among the best documented among potential human pathogens);
(6) CMV is not associated with malignancies; and most significantly;
(7) CMV is ubiquitous—most of humanity, including the vast majority of subjects in areas with high incidence HIV infection, already harbor this virus.

Given the safety issues of attenuated lentivirus vaccines, which have led to their near abandonment as potential human vaccines, the last points are very significant. CMV, as disclosed herein and as supported by the data presented below, has substantial utility for effectively functioning as a vector in subjects with prior CMV immunity, and the use of these vectors—even those based on the wildtype CMV genome (e.g., not further modified for safety)—exposes such vaccinees to little risk over and above what they are already subject to as a result of their naturally acquired CMV.

Therefore, according to particular aspects:
- CMV (*e*.*g*., HCMV) is engineerable to express determinants (*e.g*., SIV, HIV, TB) in highly immunogenic form, and to invoke high level, persistent cellular and humoral immunity in vaccinated subjects, even those that have had prior CMV exposure;
- the immunity to SIV and HIV determinants is superior in intensity, persistence, and/or "quality" (*e*.*g*., functional attributes and localization) to prior art approaches, resulting in more effective control of pathogenic challenges (*e*.*g*., SIV, HIV, TB);
- repeated vaccinations with the same core CMV-based vector containing different/modified Ags is feasible and productive, because of CMV's highly evolved capability to "slip" by the immune response, thereby allowing either the *de novo* generation of unrelated responses, or responses to new epitopes contained within a modified Ag; and
- the wildtype CMV genome is modifiable to enhance safety without sacrificing immunogenicity and persistence.

### Specific preferred embodiments:

The vector of the present invention is useful in a method for treatment or prevention of TB, comprising infection of a subject in need thereof with at least one recombinant HCMV vector comprising an expressible TB antigen or a variant or fusion protein thereof. In particular embodiments, infection is of an immunocompetent, TB seropositive subject. In certain embodiments, the at least one TB antigen is selected from the group consisting of ESAT-6, Ag85A, AG85B, MPT51, MPT64, CFP10, TB10.4, Mtb8.4, hspX, CFP6, Mtb12, Mtb9.9 antigens, Mtb32A, PstS-1, PstS-2, PstS-3, MPT63, Mtb39, Mtb41, MPT83, 71-kDa, PPE 68, LppX, and antigenic portions, variants and fusion proteins thereof. In particular embodiments, the method further comprises serial re-infection with at least one recombinant HCMV vector comprising an expressible TB antigen or a variant or fusion protein thereof. In certain embodiments, the expressible TB antigen, or variant or fusion protein thereof, of the serial re-infection vector is different than that of the initial infection vector. In some embodiments, expression is driven by an antigen encoding sequence in operable association with a promoter selected from the group consisting of a constitutive CMV promoter, an immediate early CMV promoter, an early CMV promoter and a late CMV promoter. In some embodiments, the promoter is selected from the group consisting of EF1-alpha, MIE, pp65 and gH.

Also provided is a recombinant HCMV vaccine vector, comprising an expressible TB antigen or a variant or fusion protein thereof. Preferably, the vector comprises suicide or safety means. In certain embodiments, expression is driven by an antigen encoding sequence in operable association with a promoter selected from the group consisting of a constitutive CMV promoter, an immediate early CMV promoter, an early CMV promoter and a late CMV promoter. In some embodiments, the promoter is selected from the group consisting of EF1-alpha, MIE, pp65 and gH.

Additionally provided is a pharmaceutical composition, comprising, along with a pharmaceutically acceptable carrier or excipient, a recombinant HCMV comprising an expressible TB antigen or a variant or fusion protein thereof. Preferably, the recombinant HCMV vaccine vector comprises suicide or safety means.

### Polynucleotide Compositions.

As used herein, the terms "DNA segment" and "polynucleotide" refer to a DNA. molecule that has been isolated free of total genomic DNA of a particular species. Therefore, a DNA segment encoding a polypeptide refers to a DNA segment that contains one or more coding sequences yet is substantially isolated away from, or purified free from, total genomic DNA of the species from which the DNA segment is obtained. Included within the terms "DNA segment" and "polynucleotide" are DNA segments and smaller fragments of such segments, and also recombinant vectors, including, for example, plasmids, cosmids, phagemids, phage, viruses, and the like.

As will be understood by those skilled in the art, the DNA segments of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

"Isolated," as used herein, means that a polynucleotide is substantially away from other coding sequences, and that the DNA segment does not contain large portions of unrelated coding DNA, such as large chromosomal fragments or other functional genes or polypeptide coding regions. Of course, this refers to the DNA segment as originally isolated, and does not exclude genes or coding regions later added to the segment by the hand of man.

As will be recognized by the skilled artisan, polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*e*.*g*.,, an endogenous sequence that encodes a CMV, or TB protein or a portion thereof) or may comprise a variant, or a biological or antigenic functional equivalent of such a sequence.

Polynucleotide "variants" may contain one or more substitutions, additions, deletions and/or insertions, as further described below, preferably such that the immunogenicity of the encoded polypeptide is not diminished, relative to a native CMV, or TB protein. The effect on the immunogenicity of the encoded polypeptide may generally be assessed as described herein. The term "variants" also encompasses homologous genes ofxenogenic origin.

When comparing polynucleotide or polypeptide sequences, two sequences are said to be "identical" if the sequence of nucleotides or amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, preferably 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins-Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Natl. Acad. Sci. USA 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (*i*.*e*., the window size) and multiplying the results by 100 to yield the percentage of sequence identity.

Therefore, the present invention encompasses polynucleotide and polypeptide sequences having substantial identity to the sequences disclosed herein, for example those comprising at least 50% sequence identity, preferably at least 55%, 60%, 65%, 70%, 75%, 80%, 85%, 90%, 95%, 96%, 97%, 98%, or 99% or higher, sequence identity compared to a polynucleotide or polypeptide sequence of this invention using the methods described herein, (*e*.*g*., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

In additional embodiments, the present invention provides isolated polynucleotides and polypeptides comprising various lengths of contiguous stretches of sequence identical to or complementary to one or more of the sequences disclosed herein. For example, polynucleotides are provided by this invention that comprise at least about 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc*.; 21, 22, 23, *etc*.; 30, 31, 32, *etc*.; 50, 51, 52, 53, *etc*.; 100, 101, 102, 103, *etc.;* 150, 151, 152, 153, *etc.;* including all integers through 200-500; 500-1,000, and the like.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative DNA segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

In other embodiments, the present invention is directed to polynucleotides that are capable of hybridizing under moderately stringent conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

Moreover, it will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison)

### Polypeptide Compositions

The present invention, in other aspects, provides polypeptide compositions. Generally, a polypeptide of the invention will be an isolated polypeptide (or an epitope, variant, or active fragment thereof) derived from CMV, or TB, etc. Preferably, the polypeptide is encoded by a polynucleotide sequence disclosed herein or a sequence which hybridizes under moderate or highly stringent conditions to a polynucleotide sequence disclosed herein. Alternatively, the polypeptide may be defined as a polypeptide which comprises a contiguous amino acid sequence from an amino acid sequence disclosed herein, or which polypeptide comprises an entire amino acid sequence disclosed herein.

In the present invention, a polypeptide composition is also understood to comprise one or more polypeptides that are immunologically reactive with antibodies and/or T cells generated against a polypeptide of the invention, particularly a polypeptide having amino acid sequences disclosed herein, or to active fragments, or to variants or biologically functional equivalents thereof.

Likewise, a polypeptide composition of the present invention is understood to comprise one or more polypeptides that are capable of eliciting antibodies or T cells that are immunologically reactive with one or more polypeptides encoded by one or more contiguous nucleic acid sequences contained in the amino acid sequences disclosed herein, or to active fragments, or to variants thereof, or to one or more nucleic acid sequences which hybridize to one or more of these sequences under conditions of moderate to high stringency. Particularly illustrative polypeptides comprise the CMV, and TB amino acid sequence disclosed in the Sequence Listing.

As used herein, an active fragment of a polypeptide includes a whole or a portion of a polypeptide which is modified by conventional techniques, *e.g.,* mutagenesis, or by addition, deletion, or substitution, but which active fragment exhibits substantially the same structure function, antigenicity, etc., as a polypeptide as described herein.

In certain illustrative embodiments, the polypeptides of the invention will comprise at least an immunogenic portion of a CMV antigen or a variant or biological functional equivalent thereof, as described herein. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of a protein that is recognized (i.e., specifically bound) by a B-cell and/or T-cell surface antigen receptor. Such immunogenic portions generally comprise at least 5 amino acid residues, more preferably at least 10, and still more preferably at least 20 amino acid residues of a CMV protein or a variant thereof. Certain preferred immunogenic portions include peptides in which an N-terminal leader sequence and/or transmembrane domain have been deleted. Other preferred immunogenic portions may contain a small N- and/or C-terminal deletion (*e.g*., 1-30 amino acids, preferably 5-15 amino acids), relative to the mature protein.

Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e.,* they react with the protein in an ELISA or other immunoassay, and do not react detectably with unrelated proteins). Such antisera and antibodies may be prepared as described herein, and using well known techniques. An immunogenic portion of a native CMV, or TB protein is a portion that reacts with such antisera and/or T-cells at a level that is not substantially less than the reactivity of the full length polypeptide (*e*.*g*., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native CMV, or TB protein. A polypeptide "variant," as used herein, is a polypeptide that differs from a native CMV protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g*., 1-30 amino acids, preferably 5-15 amino acids) has been removed from the N- and/or C-terminal of the mature protein.

Polypeptide variants encompassed by the present invention include those exhibiting at least about 70%, 75%, 80%, 85%, 90%, 91%, 92%, 93%, 94%, 95%, 96%, 97%, 98%, or 99% or more identity (determined as described above) to the polypeptides disclosed herein.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide (*e*.*g*., poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast, and higher eukaryotic cells, such as mammalian cells and plant cells. Preferably, the host cells employed are *E*. *coli*, yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having less than about 100 amino acids, and generally less than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. See Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, such as a known protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided. Such proteins comprise a polypeptide as described herein together with an unrelated immunogenic protein. Preferably the immunogenic protein is capable of eliciting a recall response. Examples of such proteins include tetanus, tuberculosis and hepatitis proteins (*see,* for example, Stoute et al. New Engl. J. Med., 336:86-91, 1997).

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### Pharmaceutical Compositions

It will also be understood that, if desired, the nucleic acid segment, RNA, DNA or PNA compositions that express a polypeptide as disclosed herein may be administered in combination with other agents as well, such as, *e.g*., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Formulation of pharmaceutically-acceptable excipients and carrier solutions is well-known to those of skill in the art, as is the development of suitable dosing and treatment regimens for using the particular compositions described herein in a variety of treatment regimens, including *e.g*., oral, parenteral, intravenous, intranasal, and intramuscular administration and formulation.

### Oral Delivery

In certain applications, the pharmaceutical compositions disclosed herein may be delivered via oral administration to an animal or subject. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (Mathiowitz *et al.,* 1997; Hwang *et al.,* 1998; U. S. Patent 5,641,515; U. S. Patent 5,580,579 and U. S. Patent 5,792,451). The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. A syrup of elixir may contain the active compound sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

Typically, these formulations may contain at least about 0.1 % of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. For example, a mouthwash may be prepared incorporating the active ingredient in the required amount in an appropriate solvent, such as a sodium borate solution (Dobell's Solution). Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

### Injectable Delivery

In certain circumstances it will be desirable to deliver the pharmaceutical compositions disclosed herein subcutaneously, parenterally, intravenously, intramuscularly, or even intraperitoneally as described in U. S. Patent 5,543,158; U. S. Patent 5,641,515 and U. S. Patent 5,399,363 . Solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions (U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e*.*g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, and the general safety and purity standards as required by FDA Office of Biologics standards.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The compositions disclosed herein may be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

### Nasal Delivery

In certain embodiments, the pharmaceutical compositions may be delivered by intranasal sprays, inhalation, and/or other aerosol delivery vehicles. Methods for delivering genes, nucleic acids, and peptide compositions directly to the lungs via nasal aerosol sprays has been described e.g., in U. S. Patent 5,756,353 and U. S. Patent 5,804,212 . Likewise, the delivery of drugs using intranasal microparticle resins (Takenaga *et al.,* 1998) and lysophosphatidyl-glycerol compounds (U. S. Patent 5,725,871) are also well-known in the pharmaceutical arts. Likewise, transmucosal drug delivery in the form of a polytetrafluoroetheylene support matrix is described in U. S. Patent 5,780,045.

### Liposome-, Nanocapsule-, and Microparticle-Mediated Delivery

In certain embodiments, the inventors contemplate the use of liposomes, nanocapsules, microparticles, microspheres, lipid particles, vesicles, and the like, for the introduction of the compositions of the present invention into suitable host cells. In particular, the compositions of the present invention may be formulated for delivery either encapsulated in a lipid particle, a liposome, a vesicle, a nanosphere, or a nanoparticle or the like.

Such formulations may be preferred for the introduction of pharmaceutically-acceptable formulations of the nucleic acids or constructs disclosed herein. The formation and use of liposomes is generally known to those of skill in the art (see for example, Couvreur *et al.,* 1977; Couvreur, 1988; Lasic, 1998; which describes the use of liposomes and nanocapsules in the targeted antibiotic therapy for intracellular bacterial infections and diseases). Recently, liposomes were developed with improved serum stability and circulation half-times (Gabizon and Papahadjopoulos, 1988; Allen and Choun, 1987; U. S. Patent 5,741,516). Further, various methods of liposome and liposome like preparations as potential drug carriers have been reviewed (Takakura, 1998; Chandran *et al.,* 1997; Margalit, 1995; U. S. Patent 5,567,434; U. S. Patent 5,552,157; U. S. Patent 5,565,213; U. S. Patent 5,738,868 and U. S. Patent 5,795,587).

Liposomes have been used successfully with a number of cell types that are normally resistant to transfection by other procedures including T cell suspensions, primary hepatocyte cultures and PC 12 cells (Renneisen *et al.,* 1990; Muller *et al.,* 1990). In addition, liposomes are free of the DNA length constraints that are typical of viral-based delivery systems. Liposomes have been used effectively to introduce genes, drugs (Heath and Martin, 1986; Heath *et al.,* 1986; Balazsovits *et al.,* 1989; Fresta and Puglisi, 1996), radiotherapeutic agents (Pikul *et al.,* 1987), enzymes (Imaizumi *et al.,* 1990a; Imaizumi *et al*., 1990b), viruses (Faller and Baltimore, 1984), transcription factors and allosteric effectors (Nicolau and Gersonde, 1979) into a variety of cultured cell lines and animals. In addition, several successful clinical trials examining the effectiveness of liposome-mediated drug delivery have been completed (Lopez-Berestein *et al.,* 1985a; 1985b; Coune, 1988; Sculier *et al.,* 1988). Furthermore, several studies suggest that the use of liposomes is not associated with autoimmune responses, toxicity or gonadal localization after systemic delivery (Mori and Fukatsu, 1992).

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 Å, containing an aqueous solution in the core.

Liposomes bear resemblance to cellular membranes and are contemplated for use in connection with the present invention as carriers for the peptide compositions. They are widely suitable as both water- and lipid-soluble substances can be entrapped, *i*.*e*., in the aqueous spaces and within the bilayer itself, respectively. It is possible that the drug-bearing liposomes may even be employed for site-specific delivery of active agents by selectively modifying the liposomal formulation.

In addition to the teachings of Couvreur *et al*. (1977; 1988), the following information may be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs at a characteristic phase-transition temperature and results in an increase in permeability to ions, sugars and drugs.

n addition to temperature, exposure to proteins can alter the permeability of liposomes. Certain soluble proteins, such as cytochrome c, bind, deform and penetrate the bilayer, thereby causing changes in permeability. Cholesterol inhibits this penetration of proteins, apparently by packing the phospholipids more tightly. It is contemplated that the most useful liposome formations for antibiotic and inhibitor delivery will contain cholesterol.

The ability to trap solutes varies between different types of liposomes. For example, MLVs are moderately efficient at trapping solutes, but SUVs are extremely inefficient. SUVs offer the advantage of homogeneity and reproducibility in size distribution, however, and a compromise between size and trapping efficiency is offered by large unilamellar vesicles (LUVs). These are prepared by ether evaporation and are three to four times more efficient at solute entrapment than MLVs.

In addition to liposome characteristics, an important determinant in entrapping compounds is the physicochemical properties of the compound itself. Polar compounds are trapped in the aqueous spaces and nonpolar compounds bind to the lipid bilayer of the vesicle. Polar compounds are released through permeation or when the bilayer is broken, but nonpolar compounds remain affiliated with the bilayer unless it is disrupted by temperature or exposure to lipoproteins. Both types show maximum efflux rates at the phase transition temperature.

Liposomes interact with cells via four different mechanisms: endocytosis by phagocytic cells of the reticuloendothelial system such as macrophages and neutrophils; adsorption to the cell surface, either by nonspecific weak hydrophobic or electrostatic forces, or by specific interactions with cell-surface components; fusion with the plasma cell membrane by insertion of the lipid bilayer of the liposome into the plasma membrane, with simultaneous release of liposomal contents into the cytoplasm; and by transfer of liposomal lipids to cellular or subcellular membranes, or vice versa, without any association of the liposome contents. It often is difficult to determine which mechanism is operative and more than one may operate at the same time.

The fate and disposition of intravenously injected liposomes depend on their physical properties, such as size, fluidity, and surface charge. They may persist in tissues for h or days, depending on their composition, and half lives in the blood range from min to several h. Larger liposomes, such as MLVs and LUVs, are taken up rapidly by phagocytic cells of the reticuloendothelial system, but physiology of the circulatory system restrains the exit of such large species at most sites. They can exit only in places where large openings or pores exist in the capillary endothelium, such as the sinusoids of the liver or spleen. Thus, these organs are the predominate site of uptake. On the other hand, SUVs show a broader tissue distribution but still are sequestered highly in the liver and spleen. In general, this *in vivo* behavior limits the potential targeting of liposomes to only those organs and tissues accessible to their large size. These include the blood, liver, spleen, bone marrow, and lymphoid organs.

Targeting is generally not a limitation in terms of the present invention. However, should specific targeting be desired, methods are available for this to be accomplished. Antibodies may be used to bind to the liposome surface and to direct the antibody and its drug contents to specific antigenic receptors located on a particular cell-type surface. Carbohydrate determinants (glycoprotein or glycolipid cell-surface components that play a role in cell-cell recognition, interaction and adhesion) may also be used as recognition sites as they have potential in directing liposomes to particular cell types. Mostly, it is contemplated that intravenous injection of liposomal preparations would be used, but other routes of administration are also conceivable.

Alternatively, the invention provides for pharmaceutically-acceptable nanocapsule formulations of the compositions of the present invention. Nanocapsules can generally entrap compounds in a stable and reproducible way (Henry-Michelland *et al.,* 1987; Quintanar-Guerrero *et al.,* 1998; Douglas *et al.,* 1987). To avoid side effects due to intracellular polymeric overloading, such ultrafine particles (sized around 0.1 µm) should be designed using polymers able to be degraded *in vivo*. Biodegradable polyalkyl-cyanoacrylate nanoparticles that meet these requirements are contemplated for use in the present invention. Such particles may be are easily made, as described (Couvreur *et al.,* 1980; 1988; zur Muhlen *et al.,* 1998; Zambaux *et al.* 1998; Pinto-Alphandry *et al.,* 1995 and U. S. Patent 5,145,684, specifically incorporated herein by reference in its entirety).

### Vaccines

In certain preferred embodiments of the present invention, HCMV -based recombinant vaccines are provided. The vaccines will generally comprise one or more pharmaceutical compositions, such as those discussed above, in optional combination with an immunostimulant. An immunostimulant may be any substance that enhances or potentiates an immune response (antibody and/or cell-mediated) to an exogenous antigen. Examples of immunostimulants include adjuvants, biodegradable microspheres (*e.g*., polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other CMV antigens may be present, either incorporated into a fusion polypeptide or as a separate compound, within the composition or vaccine.

Illustrative vaccines may contain recombinant HCMV DNA encoding one or more of the polypeptides as described above, such that the polypeptide is generated *in situ.* Appropriate nucleic acid expression systems contain the necessary DNA sequences for expression in the patient (such as a suitable promoter and terminating signal). In a preferred embodiment, the DNA may be introduced using a HCMV expression system, which may involve the use of attenuated or altered CMV vectors. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells. It will be apparent that a vaccine may comprise both a polynucleotide and a polypeptide component. Such vaccines may provide for an enhanced immune response.

It will be apparent that a vaccine may contain pharmaceutically acceptable salts of the polynucleotides and polypeptides provided herein. Such salts may be prepared from pharmaceutically acceptable non-toxic bases, including organic bases (*e.g.*, salts of primary, secondary and tertiary amines and basic amino acids) and inorganic bases (*e.g*., sodium, potassium, lithium, ammonium, calcium and magnesium salts).

While any suitable carrier known to those of ordinary skill in the art may be employed in the vaccine compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g., polylactate polyglycolate) may also be employed as carriers for the pharmaceutical compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344 and 5,942,252. Modified hepatitis B core protein carrier systems are also suitable, such as those described in WO/99 40934, and references cited therein. One may also employ a carrier comprising the particulate-protein complexes described in U.S. Patent No. 5,928,647, which are capable of inducing a class I-restricted cytotoxic T lymphocyte responses in a host.

Such compositions may also comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, bacteriostats, chelating agents such as EDTA or glutathione, adjuvants (*e*.*g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate. Compounds may also be encapsulated within liposomes using well known technology.

Any of a variety of immunostimulants may optionally be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the context of the HCMV-based vaccine vectors provided herein, the adjuvant composition is optional, but if included, is preferably designed to induce an immune response predominantly of the Th1 type. High levels of Th1-type cytokines (*e.g*., IFN-γ, TNFα, IL-2 and IL-12) tend to favor the induction of cell mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Corixa Corporation (Seattle, WA; *see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant is a saponin, preferably QS21 (Aquila Biopharmaceuticals Inc., Framingham, MA), which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprise an oil-in-water emulsion and tocopherol. A particularly potent adjuvant formulation involving QS21, 3D-MPL and tocopherol in an oil-in-water emulsion is described in WO 95/17210.

Other preferred adjuvants include Montanide ISA 720 (Seppic, France), SAF (Chiron, California, United States), ISCOMS (CSL), MF-59 (Chiron), the SBAS series of adjuvants (e.g., SBAS-2 or SBAS-4; available from SmithKline Beecham, Rixensart, Belgium), Detox (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720. Other preferred adjuvants comprise polyoxyethylene ethers, such as those described in WO 99/52549A1.

Any vaccine provided herein may be prepared using well known methods that result in a combination of vector, optional immune response enhancer and a suitable carrier or excipient. The compositions described herein may be administered as part of a sustained release formulation (*i*.*e*., a formulation such as a capsule, sponge or gel (composed of polysaccharides, for example) that effects a slow release of compound following administration). Such formulations may generally be prepared using well known technology (*see, e.g.,* Coombes et al., Vaccine 14:1429-1438, 1996) and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane.

Carriers for use within such formulations are biocompatible, and may also be biodegradable; preferably the formulation provides a relatively constant level of active component release. Such carriers include microparticles of poly(lactide-co-glycolide), polyacrylate, latex, starch, cellulose, dextran and the like. Other delayed-release carriers include supramolecular biovectors, which comprise a non-liquid hydrophilic core (*e.g.,* a cross-linked polysaccharide or oligosaccharide) and, optionally, an external layer comprising an amphiphilic compound, such as a phospholipid (*see e.g.,* U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets CMV-infected cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-CMV effects *per se* and/or to be immunologically compatible with the receiver (*i*.*e*., matched HLA haplotype). APCs may generally be isolated from any of a variety of biological fluids and organs and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic immunity (*see* Timmerman and Levy, Ann. Rev. Med. 50:507-529, 1999). In general, dendritic cells may be identified based on their typical shape (stellate *in situ,* with marked cytoplasmic processes (dendrites) visible *in vitro),* their ability to take up, process and present antigens with high efficiency and their ability to activate naive T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g*., CD40, CD80, CD86 and 4-1BB).

APCs may generally be transfected with a polynucleotide encoding a CMV, or TB protein (or portion or other variant thereof) such that the CMV, or TB polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the CMV polypeptide, DNA (naked or within a plasmid vector) or RNA; or with antigen-expressing recombinant bacterium or viruses (*e*.*g*., vaccinia, fowlpox, adenovirus or lentivirus vectors). Prior to loading, the polypeptide may be covalently conjugated to an immunological partner that provides T cell help (*e*.*g*., a carrier molecule). Alternatively, a dendritic cell may be pulsed with a non-conjugated immunological partner, separately or in the presence of the polypeptide.

Vaccine vectors and pharmaceutical compositions may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a vaccine or pharmaceutical composition may be stored in a freeze-dried condition requiring only the addition of a sterile liquid carrier immediately prior to use.

### Immunotherapeutic Applications

In further aspects of the present invention, the compositions described herein may be used for immunotherapy of TB infections. Within such methods, pharmaceutical compositions and vaccines are typically administered to a patient. As used herein, a "patient" refers to any warm-blooded animal, preferably a human. The above pharmaceutical compositions and vaccines may be used to prophylactically prevent or ameliorate the extent of infection by TB or to treat a patient already infected with TB. Administration may be by any suitable method, including administration by intravenous, intraperitoneal, intramuscular, subcutaneous, intranasal, intradermal, anal, vaginal, topical, and oral routes.

Within preferred embodiments, immunotherapy is active immunotherapy, in which treatment relies on the *in vivo* stimulation of the endogenous host immune system to react against TB infection with the administration of immune response-modifying agents (such as polypeptides and polynucleotides as provided herein).

Routes and frequency of administration of the therapeutic compositions described herein, as well as dosage, will vary from individual to individual, but may be readily established using standard techniques. In one embodiment, between 1 and about 10 doses may be administered over a 52 week period. In another embodiment, about 6 doses are administered, at intervals of about 1 month, and serial vaccinations are opotionally given periodically thereafter. Alternate protocols may be appropriate for individual patients.

A suitable dose is an amount of a compound that, when administered as described above, is capable of promoting an anti-TB immune response, and is preferably at least 10-50% above the basal (i.e., untreated) level. Such response can be monitored, for example, by measuring the anti-TB antibodies in a patient. Such vaccines should also be capable of causing an immune response that leads to an improved clinical outcome (*e*.*g*., more frequent remissions, complete or partial or longer disease-free survival) in vaccinated patients as compared to non-vaccinated patients. In general, for pharmaceutical compositions and vaccines comprising one or more polypeptides, the amount of each polypeptide sought in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to a TB protein may correlate with an improved clinical outcome. Such immune responses may generally be evaluated using standard proliferation, cytotoxicity or cytokine assays, which may be performed using samples obtained from a patient before and after treatment.

### EXAMPLE 1

### (CMV was demonstrated to have utility as a vaccine vector for prevention and/or treatment of, TB)

### RATIONALE/PREMISE:

*Unique Characteristics of HIV*/*SIV Infection.* HIV and its non-human primate (NHP) counterpart SIV share a pattern of infection and a constellation of pathobiologic features that in the vast majority of susceptible hosts results in unremitting infection, progressive immunodeficiency, and ultimately, death (1-4). This progression occurs despite clear-cut cellular and humoral anti-viral immune responses (5, 6), and clinical situations consistent with immune control of untreated HIV/SIV infection are relatively unusual, difficult to confirm as immunologic in nature, and more often than not, impermanent 7-12). Several fundamental features of the virus likely contribute to the relentless, immune "resistant" nature of these infections. *First,* HIV/SIV infect the immune system and by both direct and indirect mechanisms, progressively disrupt its physiology (4, 13-17). The clinical hallmark of HIV/SIV infection is, of course, CD4+ T cell depletion, but the total effect of these viruses on the immune system is pervasive, ultimately affecting the function of almost all components of the system. Moreover, these effects are cumulative and reinforcing; they initiate early in infection and eventuate in a progressive spiral of immune dysfunction (16). The pathophysiology, in addition to loss of peripheral CD4+ T cells and its extended effects on CD8+ T cell and B cell function, HIV/SIV induces, *inter alia,* a generalized state of hyperactivation with attendant deleterious effects on both CD4+ and CD8+ T cell homeostasis and function, thymocyte infection and thymus destruction, macrophage infection and associated damage/dysfunction of peripheral immune microenvironments (14, 17). The inevitable end result of sustained viral replication is therefore progressive degradation of immune capabilities including, most notably, the ability of the anti-viral immunity to control the virus itself (18-20).

Immune control of HIV/SIV is also impeded by a *second* feature of these viruses— specific evolutionary adaptations to avoid neutralizing antibody responses, including 1) concealing conserved, functionally important structures within the interior of the prefusogenic env glycoprotein trimers, 2) hiding them by glycosylation, 3) using conformation flexibility to undermine the development of high affinity Abs to discontinuous, potentially neutralization-sensitive epitopes, and 4) decoy immunogenicity (directing most of the antibody response to irrelevant epitopes) (21, 22). Thus, despite the fact that neutralizing antibody can in certain experimental circumstances protect against viral challenge (see below), in most natural infections the antibody response that develops is thought to be relatively ineffective.

*Third,* the high replication capability and genetic mutability of these lentiviruses allows their rapid response to almost any selection pressure, increasing adaptation to changing host conditions, and resulting in viral variants with enhanced pathogenicity (23-26), or diminished susceptibility to anti-viral immunity (27-31). Thus, unless an immune response is directed at a functionally critical, invariant segment of a viral gene product, simultaneously targets a broad array of epitopes with potent effector activity, and/or is present early enough in infection so as to precede the explosive population growth and production of viral variants upon which selection operates, the high replication and mutation rate of HIV/SIV will ensure the outgrowth of a sufficiently fit viral variant so as to maintain progressive infection. In most infections, conditions for immune control are not met, and the virus appears to rapidly escape those effective responses that do arise.

*Finally,* these viruses can achieve true latency—long-term, genetically silent infection with the potential for re-activation and full expression of their infectious program (32, 33). The existence of such a latent pool of genetically diverse viral genomes suggests that at this point of understanding, cure of HIV/SIV is almost certainly not achievable, and that an apparently controlled infection always has the potential for re-ignition and renewed pathogenicity with any decline in activity of the controlling mechanisms.

*Impact of Immunity to HIV*/*SIV During Natural Infection Provides Rationale For Aspects of the Present Invention.* The question is, therefore, not whether immune responses to HIV/SIV develop—they certainly do (5, 6, 22, 34, 35)—but rather, whether such responses impact viral replication and disease progression. Definitive immune correlates of protection in slowly progressive vs. rapidly progressive disease have been difficult to identify, and even when associations are confirmed, there is the issue of cause and effect in an immunosuppressive virus; that is, does a strong immune response control the virus, or does a non-aggressive infection due to virologic or non-immune host factors simply lead to better preservation of the anti-HIV immune response (9)? The overarching question is whether any general realization of immune control of viral replication with lifelong arrest of disease progression is achievable. Given the difficulty in finding clear-cut examples of immunity in natural infection, and the impressive capability of these viruses to evade immunity, our chances would appear to be slim. However, new lines of evidence, primarily in the SIV-rhesus macaque (RM) experimental model, have more convincingly supported the concept of "effective" anti-lentivirus immunity. Indeed, the evidence cited above for rapid immune escape in SIV infection—the specific modification of the infecting cloned virus in those parts of its genome encoding recognized CTL epitopes (27, 29)— is fundamentally *prima facie* evidence of effective (albeit, not protective) immunity. For immune escape to occur, the SIV-specific, CD8+ T cell response in question must have successfully suppressed the original epitope-bearing viral species. Evidence for such immune-mediated viral adaptation has also been found in human HIV infection (36), and these data, when combined with strong HLA class I allele correlations with disease progression (37, 38), make a persuasive case for CD8+ T cell function in HIV infection. The ongoing participation of CD8+ T cells in determination of viral replication levels is also supported by data in the RM model indicating enhancement of SIV replication following mAb-mediated CD8+ cell depletion, including during primary infection, chronic infection, and the period of viral control following transient post-inoculation anti-retroviral treatment (39-43). Even more convincing is the emerging data demonstrating the ability of newer vaccine protocols, which are largely focused on the generation of CTL responses, to protect against pathogenic challenge with certain SIV strains (44-60). While these data, discussed in more detail below, need to be interpreted in the context of the specific challenge model used, they provide 'proof of principle' that a strong cellular immune response can control these viruses for extended periods of time.

Finally, there is the question of humoral immunity. Genetic changes in the env gene which result in neutralization escape appear to occur in HIV/SIV infection (22, 61), but given the frequency of env modifications in these viruses and the fact that env structure controls other aspects of pathogenicity (*e*.*g*., the nature of 'target' cell populations; 62), it is difficult to determine whether these changes reflect actual immune selection. Several groups have reported successful prophylaxis of SIV/HIV hybrid (SHIV) infection with immediate post-challenge treatment with neutralizing Abs (3-66), but such treatment has not been shown to be effective in established infections (67), perhaps due to cell-to-cell transmission of the virus. Thus, at this point, there remains consensus that a strong 'pre-positioned' (*e.g.,* vaccine-induced) neutralizing antibody response could significantly contribute to the critical initial control of infecting virus, but means by which such responses might be reliably generated remained to be determined; that it, until aspects of the present invention.

*SIV*/*SHIV vaccines—Prior Progress.* Efforts to develop a prophylactic AIDS vaccine commenced with the discovery of HIV in 1983-84. However, early failures of vaccine strategies based on conventional approaches (*e.g.,* primarily designed to generate antibody responses, before the complexities of env structure and function were known) sent the field 'back to the drawing board' (68-71). The isolation of SIV from monkeys with AIDS-like illness in 1985, and the subsequent development of SIV and SHIV models in various macaque species offered a viable alternative to expensive and slow clinical studies (52, 72, 73) and the field has largely accepted 'proof of principle' studies in the SIV/macaque model to both define the basic immunologic features of lentiviral infection, and to provide a rapid testing ground for various vaccine strategies. The SHIV (hybrid) models were especially attractive, as the use of HIV env seemed to offer more relevance to HIV infection in humans. A number of vaccine approaches, including attenuated (*e.g.,* gene segment-deleted) SIVs, DNA alone, DNA and replication-deficient vaccinia *(e.g.,* MVA) or adenovirus vectors, or viral vectors alone (vaccinia, adenovirus, vesicular stomatitis virus) (the DNAs and non-SIV vectors encoding various SIV or HIV open reading frames (ORFs)), have elicited cellular (± humoral) immune responses that have been associated with viremia control and protection from disease in such SHIV challenges (44-51). While these studies have demonstrated the central importance of cellular immunity, specifically CD8+ CTL, in effective anti-viral responses, and have provided the 'proof of principle' that immunologic control of some model lentiviral infections is possible, their relevance to HIV infection has recently been questioned (74). To date, pathogenic SHIVs are CXCR4-utilizing viruses (most SIVs and transmitted HIVs are CCR5-utilizing), and the SHIV89.6P virus most commonly used as a vaccine model elicits rapid CD4+ T cell depletion (likely because its CXCR4 co-receptor utilization allows it to infect/destroy both naïve and memory T cells, as opposed to the memory T cell tropism of CCR5-utilizing viruses), and causes the acute onset of an AIDS-like illness and early death (not the chronic progressive course of typical AIDS/SAIDS). It has been noted that SHIV89.6P is unusually sensitive to antibody neutralization, and that animals that survive the acute pathogenicity of SHIV89.6P infection often manifest a relatively benign course (74, 75) (even without vaccination; L. Picker and M. Axthelm, personal observations). Thus, vaccines that exert control during acute infection, preventing the initial, dramatic CD4+ T cell depletion of acute SHIV89.6P infection, may allow for the development of a uniquely protective antibody response which would then mediate long-term protection with this challenge (with occasional viral escape and renewed pathogenicity (76). Although the tempo of progression of pathogenic SIV infection (SIVmac239, mac251, smE660) is accelerated as compared to HIV infection of human (52), the chronic, progressive pathogenicity of these CCR5-utilizing viruses is thought to more closely reflect that of HIV (74, 75). Challenge models using these viruses have shown some efficacy for several of the vaccine approaches mentioned above (52-60), although a recent study has shown that while high level T cell immunity generated by a DNA/MVA prime boost protocol significantly blunted the viremia of primary SIVmac239 infection, by 12 weeks post infection, viral replication of the vaccinated animals was no different from that of the controls (75). It should also be noted that most challenges are identical or substantially homologous to the SIV components of the vaccine, and sequence heterogeneity in field strains of HIV will likely reduce the effectiveness of current vaccine strategies even further (77).

Significantly, among the myriad vaccine strategies tested in the SIV models to date, the strategy with the best track record for containment of highly pathogenic and even heterologous challenge in diverse NHPs is the 'gene segment-deleted' attenuated SIV approach (51, 78-82, 134). Unfortunately, the effectiveness of these attenuated SIV vaccines appears to directly correlate with their potential for *in vivo* growth and pathogenicity (*e.g.,* modest attenuation is optimal; increasing attenuation undermines protection), raising serious safety issues and undermining the viability of prior art attenuation strategies for production of human HIV vaccines (79, 83). Nevertheless, the ability of attenuated SIV infections to elicit such effective protection against RS-tropic, chronic-aggressive SIV challenge offers lessons for development of alterative vaccine strategies. While it is not possible to completely rule out a role for viral interference, innate immunity, or other virus-induced changes in target cell number or susceptibility that undermines the establishment of the pathogenic challenge (86-89), recent evidence strongly suggests that the induction and maturation of humoral (nAb) and cellular (CTL) anti-SIV immune responses correlates with their efficacy (Schmitz, J. et al., Abstract #15, 21st Annual Symposium on NHP Models for AIDS, October 24, 2003). The inability of other vaccine strategies (*e*.*g*., DNA, heterologous vectors; prime-boost protocols) to recapitulate the efficacy of attenuated SIV vaccines (despite having as large or larger T cell response frequencies) suggests there might be differences in the 'quality' of the responses that are elicited by these approaches.

*Importance of chronicity and other factors.* One obvious potential mechanism for such differences is the chronicity of exposure of the vaccinee immune system to Ag, a potential requirement for the generation, maturation and maintenance of a quantitatively and functionally appropriate immune response against a persistent, elusive lentivirus. The observation that attenuated SIV infections appear to require weeks to months for protective responses to mature (134) is in keeping with this idea. Non-replicating viral vectors or vectors that are ultimately eliminated or highly contained by anti-vector immune responses would not be expected to provide such sustained exposure. Other possibilities include: 1) the route of Ag exposure or location(s) of vector infection (*e.g.,* current vectors might not provide sufficient mucosal immunity), or 2) the adjuvant characteristics of the vector (*e.g.,* the provision of regulatory signals that provoke an appropriate mix of effector responses). The bottom line is that current approaches, though improving, might lack key characteristics that will be necessary for a 'field-effective' HIV vaccine, warranting serious consideration of alternative approaches.

Therefore, according to aspects of the present invention, and as will be discussed in the following sections, the β-herpesvirus CMV possesses unique immunogenicity and virologic characteristics that provide substantial utility as a vaccine vector for persistent pathogens such as SIV/HIV, TB, etc.

*Pathobiology and Immunology of CMV Infection.* Cytomegaloviruses (CMVs) comprise a distinct, widely distributed subgroup of β-herpesviruses that share common growth characteristics, characteristic cytopathology, salivary gland tropism and a capacity to establish persistent and latent infection (90). Among the largest and most complex of known viruses, CMV virions range in size from 150-200 nm and include a double stranded DNA genome of 230kb—capable of coding for more than 200 proteins (90). The β-herpes viruses in general, and the CMVs in specific, are thought to have emerged prior to mammalian radiation, and therefore, viral evolution has accompanied mammalian speciation such that each species has their own uniquely adapted CMV, and CMV relatedness generally parallels species relatedness. Thus, the genetics and biology of primate CMVs (human, chimp, RM) are considerably more closely related to each other than to rodent CMVs (see below). In both humans in developing counties and in RM, CMV infection is essentially universal with 90-100% of adults showing serologic evidence of infection (91-93). In affluent areas of developed countries, increased hygiene has somewhat limited transmission with only 40-60% of the adult population showing seroreactivity. For the vast majority of exposed (immunologically normal) subjects, either human or monkey, acute infection with CMV is completely asymptomatic (91, 94, 95); a small fraction of humans (<5%) experience symptomatic, but benign illness, and an even smaller fraction experience a mononucleosis syndrome (CMV accounts for ~8% of all cases of mononucleosis) (91, 94). After initial infection, CMV is shed for months to years in multiple body fluids (saliva, tears, urine, genital secretions, breast milk), and transmission generally involves mucosal exposure to such fluids, and not surprisingly occurs in situations where such secretion 'transfer' is common (early childhood and adolescence) (91, 96, 97). CMV persists in the host indefinitely and viral shedding can occur years after exposure. CMV can manifest latent infection of its target cells (90, 98), and like other herpes viruses, this capability likely plays a role in this remarkable persistence. However, unlike herpes simplex and varicella-zoster virus, the frequency of shedding (particularly in monkeys (97)), the kinetics of reactivation after transplantation (99), and the unique strength of the T cell response (see below) suggest that foci of active CMV replication are frequently, if not continuously, occurring somewhere in the infected host's body. Such active persistence implies a means for evading host immunity, and indeed, CMV has evolved diverse mechanisms for manipulating both innate and adaptive immune responses, including genes that modulate/interfere with 1) Ag presentation and other major histocompatibility complex (MHC) protein function, 2) leukocyte migration, activation and cytokine responses, 3) Fc receptor function, and 4) host cell susceptibility to apoptosis induction 100-105). These sophisticated immune evasion strategies might explain CMV's ability to re-infect immune hosts (106, 107, and see below).

Despite this impressive persistence and immune evasion, and despite CMV's ability to replicate in a wide variety of cell types (90), overt disease in chronic CMV infection is exceedingly rare. Indeed, given its essentially apathogenic nature in normal children and adults, CMV would likely be an obscure virus, if not for the discovery in the 1950s of its involvement with fetal/neonatal cytomegalic inclusion disease—an important medical problem because of its associated damage to the CNS and sensory organs—and later, its appearance as one of the most common and devastating opportunistic pathogens in the settings of organ/bone marrow transplantation and AIDS (91). These situations have one striking commonality— immunodeficiency, particularly in cell-mediated immunity, related to immunologic immaturity, pharmacologic immunosuppression (transplantation), or the progressive immunodeficiency of HIV infection. This well-characterized relationship between immunity and CMV disease suggests that CMV infection exemplifies a complex host-parasite relationship in which a delicate balance has been evolutionarily 'negotiated' between viral mechanisms of pathogenesis, persistence, and immune evasion and the host immune response. In essence, the virus is not eliminated from the host and has relatively free rein to replicate in excretion sites; yet host immunity restricts replication in most sites—those in which such replication would lead to disease. With this balance, the virus enjoys pervasiveness in a large host population that could not occur with unchecked pathogenicity. As for the host, the ability of a normal immune system to essentially eliminate pathogenicity within reproductively relevant members of the population suggests that the support of this 'pathogen' does not pose a significant evolutionary handicap. It should be acknowledged that this relationship is not without peril for even immunologically normal hosts. CMV infection has been suggested to be a risk factor for atherosclerosis, and although it is neither necessary nor sufficient for the development of this condition, there is growing evidence that it might be one contributing factor, of many, to the development of this vascular disease (91).

### RESULTS (see also Example 2 herein below)

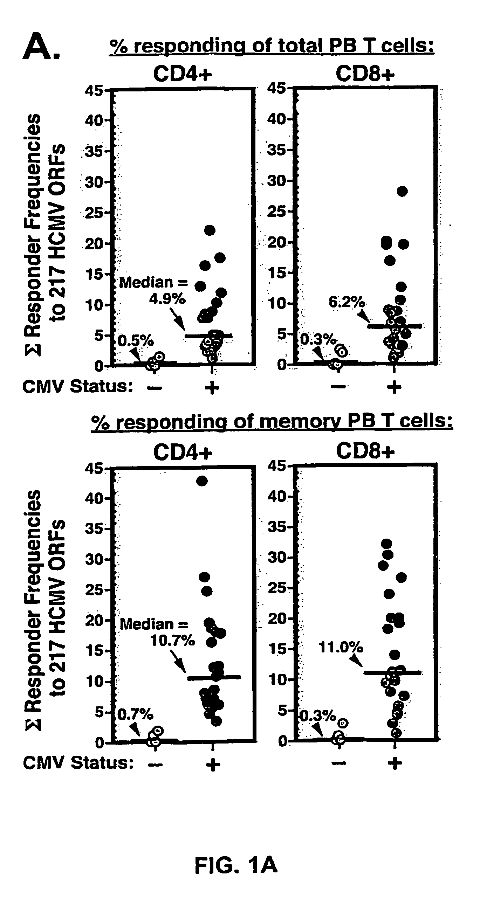

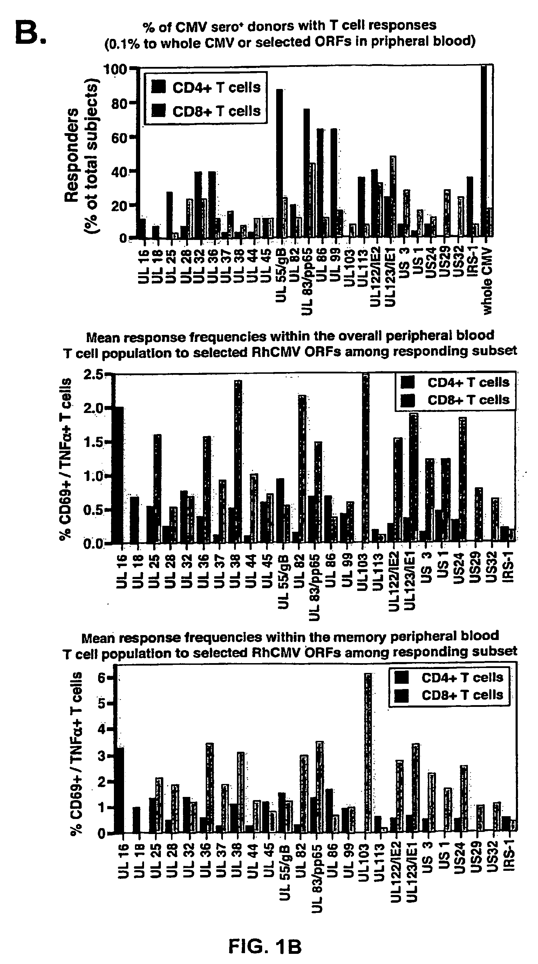

Although the immunologic requirements for maintaining this host-virus balance are not yet well characterized, the immunologic 'resources' specifically devoted to CMV are known to be quite large. The median CD4+ T cell response frequencies to human CMV (HCMV) are 5-10 fold higher than the median CD4+ T cell response frequencies to (whole) non-persistent viruses such as mumps, measles, influenza, and adenovirus, and even other persistent viruses such as herpes simplex and varicella-zoster viruses (34, 108-114). High frequency CD8+ T cell responses to particular HCMV epitopes or ORFs are also typical (115-118). Applicants have quantified total blood CD4+ and CD8+ T cell responses to the entire CMV genome (using ~14,000 overlapping peptides, cytokine flow cytometry, and a large cohort of HLA-disparate CMV-seropositive subjects). To date, applicants have 'interrogated' all of these peptides (217 ORF mixes) in 24 CMV sero+ and 5 sero- subjects and have found that when totaled, the median frequencies of CMV-specific CD4+ and CD8+ T cells in CMV-sero+ subjects are 5-6% for the total CD4+ or CD8+ T cell populations (which corresponds to 10-11% of the memory populations) (FIGURE 1) (The total responses in sero- subjects are less than 0.5%, a number that represents the 'background' of this summation analysis). Astonishingly, in some individuals, CMV-specific T cells account for more than 25% of the memory T cell repertoire in peripheral blood. This T cell response is broad—each subject recognizes an average of 19 HCMV ORFs (~ equally distributed among CD4 and CD8 responses), and at least 28 ORFs provoke significant CD4+ or CD8+ T cell responses (≥ 0.2% of peripheral blood memory population) in 20% or more of the subjects tested. The precise mechanisms by which these large CMV-specific T cell populations are generated and maintained following infection are not understood, but probably relate to the ability of this virus to maintain infection, including active, productive infection in local microenvironments, in the face of a substantial immune response over the life of the host. In addition, CMV is capable of re-infecting fully immune hosts (106, 107), and applicants have shown in the RM model (see below) that experimental re-infection significantly increases steady state frequencies of CMV-specific T cells, indicating that periodic re-infection plays a central role in the extraordinary build-up of CMV-specific T cell response frequencies. Finally, the abilities of HCMV to infect professional Ag-presenting cells (macrophages, dendritic cells) (119), and produce abundant dense bodies (enveloped tegument protein complexes that can 'infect' target cells but lack genetic material) (90) might contribute to the generation of these robust T cell responses.

Importantly, the characteristics of the antiviral antibody response to HCMV mirror those mentioned above for antiviral T cell immunity. HCMV-specific antibody responses are notable for their reactivity with a large number of viral proteins and by the persistence of stable titers against viral proteins for decades (120-123). Antibodies specific for HCMV-encoded proteins, including those against structural proteins made only during virus replication, develop rapidly after primary infection and are maintained at significant titers, likely because of the persistent expression of virus-encoded proteins (120, 124). Antibody specific for HCMV is also present on mucosal surfaces, perhaps as a result of the tropism of this virus for secretory glands. Interestingly, anti-HCMV antibody responses also boost following both community-acquired re-infections and re-infections in transplant recipients.

*The rhesus macaque (RM) model of CMV infection.* As indicated above, RM, the most utilized animal model of lentivirus (SIV) infection (52, 72, 125), display a natural CMV infection that closely mimics human infection with HCMV in terms of epidemiology, patterns of infection and disease in immunocompetent and immunodeficient hosts, particularly including RhCMV's role as a major opportunistic pathogen of SIV-infected monkeys (95, 126-131). Not surprisingly, this biologic relatedness is reflected by genetic relatedness. Early work revealed high homology between the HCMV genes gB, IE1 and UL121-117 and their RhCMV homologues (132, 133). Applicants recently obtained and analyzed the complete sequence of RhCMV strain 68.1, and identified 236 potential ORFs of 100 or more amino acids that are positionally arranged in similar fashion as their HCMV counterparts. Of these 236 ORFs, 138 (58.47%) are clearly homologous to known HCMV proteins. Some of the RhCMV that are homologous to HCMV orfs known to be nonessential for replication in fibroblasts are listed in TABLE 1 below. These orfs, according to aspects of the present invention are preferred sites for insertion of pathogenic antigens to for expression in the inventive CMV-based vaccine vectors.

In comparison, murine CMV encodes 170 ORFS, of which 78 (45.9%) are homologous to known HCMV proteins. Importantly, in contrast to murine CMV, RhCMV encodes a full complement of HCMV-like immune evasion genes, and tegument proteins with sufficient homology to HCMV to form dense bodies.

**TABLE 1. Representative examples ofRhCMV ORFs with homologues in the HCMV genome. Each annotated RhCMV ORF, as well as several previously unrecognized ORFs (Rh35a and Rh160a), was compared to the full set of HCMV ORFs using the BlastP algorithm. Scores with a significance of ≤10⁻⁵ were considered matches. If more than one RhCMV ORF corresponded to an HCMV ORF (e.g., Rh111 and 112 are homologous to UL83) the RhCMV ORF was excluded from the list. Nine of the RhCMV ORFs have been mutated by insertion of a transposon, and insert sites are indicated. The Rh151/2 ORFs correspond to the spliced HCMV UL118/9 ORFs.**

| **RhCMV orfs** | **HCMV orfs** | **RhCMV mutant** |
|---|---|---|
| Rh01 | RL1 | |
| Rh05 | UL153 | |
| Rh17 | UL4 | |
| Rh19 | UL7 | |
| Rh20 | UL6 | |
| Rh31 | UL13 | |
| Rh33 | UL14 | 25698, 25739 |
| Rh35a | UL19 | |
| Rh36 | UL20 | |
| Rh40 | UL23 | 31242 |
| Rh42 | UL24 | 31782, 32619 |
| Rh43 | UL25 | 33323,33711 |
| Rh54 | UL31 | |
| Rh56 | UL33 | |
| Rh59 | UL35 | |
| Rh68 | UL42 | |
| Rh69 | UL43 | 54274 |
| Rh72 | UL45 | 57859, 58210 |
| Rh107 | UL78 | |
| Rh123 | UL88 | 122332, 122472 |
| Rh143 | UL111A | |
| Rh148 | UL116 | |
| Rh151/2 | UL118/9 | |
| Rh155 | UL121 | 155860 |
| Rh158 | UL147 | |
| Rh159 | UL148 | 165110 |
| Rh160 | UL132 | |
| Rh160a | UL130 | |
| Rh162 | UL145 | |
| Rh163 | UL144 | |
| Rh164 | UL141 | |
| Rh181 | US1 | |
| Rh182 | US2 | |
| Rh184 | US3 | |
| Rh189 | US11 | |
| Rh190 | US12 | |
| Rh192 | US13 | |
| Rh198 | US17 | |
| Rh199 | US18 | |
| Rh200 | US19 | |
| Rh201 | US20 | |
| Rh202 | US21 | |
| Rh203 | US22 | |
| Rh221 | US29 | |
| Rh223 | US30 | |
| Rh225 | US31 | |

Basically, BlastP was used to search the RhCMV genome for ORFs that are homologous to HCMV ORFs known to be nonessential for replication in fibroblasts. These nonessential HCMV ORFs were identified: (i) from the literature; (ii) from transposon mutagenesis of the AD169 strain of HCMV; and (iii) from the fact that they are in clinical isolates but not the AD169 laboratory strain. A total of 48 RhCMV ORFs met these criteria and they are listed in TABLE1 . The HCMV homologues of three of these ORFs (Rh182, 184 and 189) are known to be immuno-modulatory genes, and three additional immunomodulatory ORFs (Rh185, Rh186 and Rh187) were not identified in applicants' BlastP analysis because their HCMV homologues were deleted during the BAC cloning of the clinical HCMVs that were sequenced. To confirm the relationship between the RhCMV and HCMV ORFs, ClustalW was used to perform multiple sequence alignments. Each RhCMV ORF was compared to the corresponding ORFs from four clinical isolates of HCMV . This analysis demonstrated that each RhCMV ORF has an orthologue in all four HCMV clinical isolates that have been sequenced, and it confirmed that the ORFs from the rhesus and human viruses are indeed related, ruling out the possibility that the original BlastP scores resulted from short homologies in otherwise unrelated proteins.

In particular exemplary vectors, two gene blocks are deleted: Rh182-189, which contains homologues to known HCMV immuno-modulatory genes, and Rh158-166, which contains genes that are present in clinical isolates but missing in laboratory strains of HCMV.

RhCMV ORFs that lack an HCMV homologue, or that correspond to an HCMV gene that is known to be essential or to augment replication in cultured fibroblasts are, according to the present invention disfavored for purposes of RhCMV and HCMV vaccine vector design.

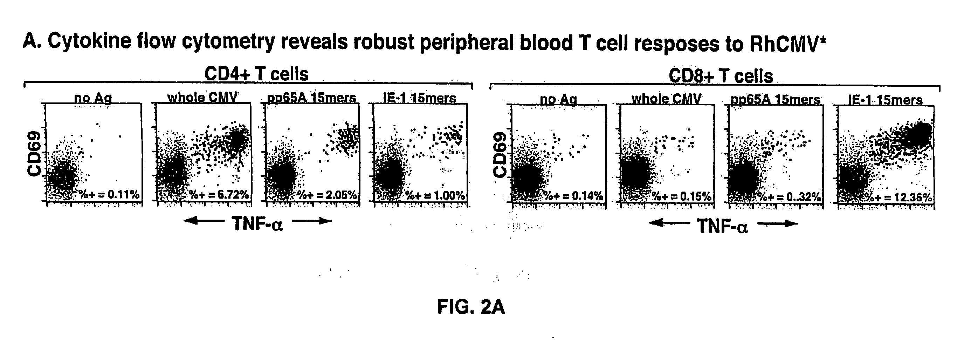

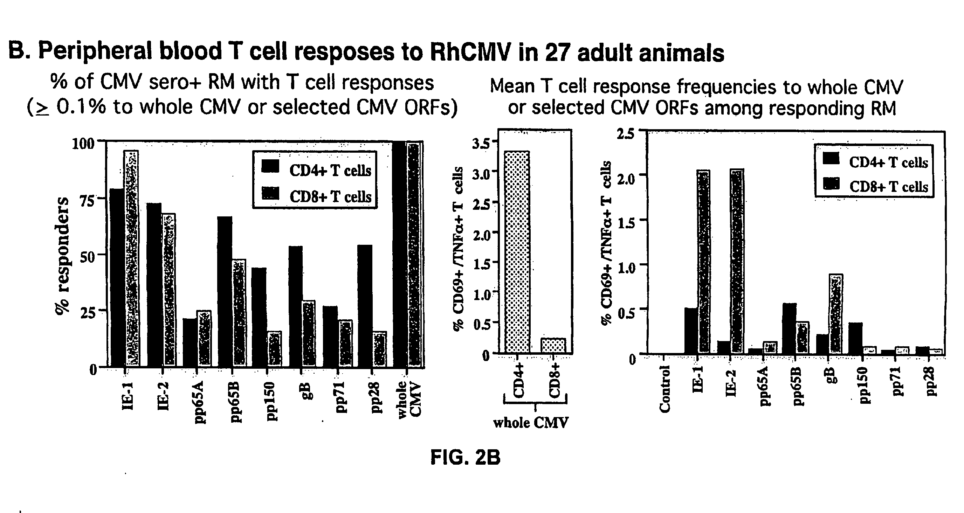

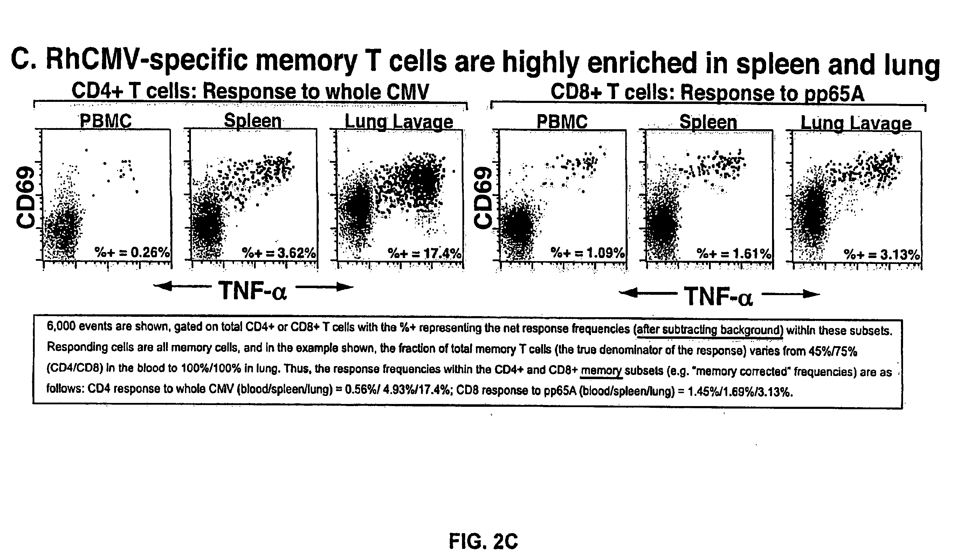

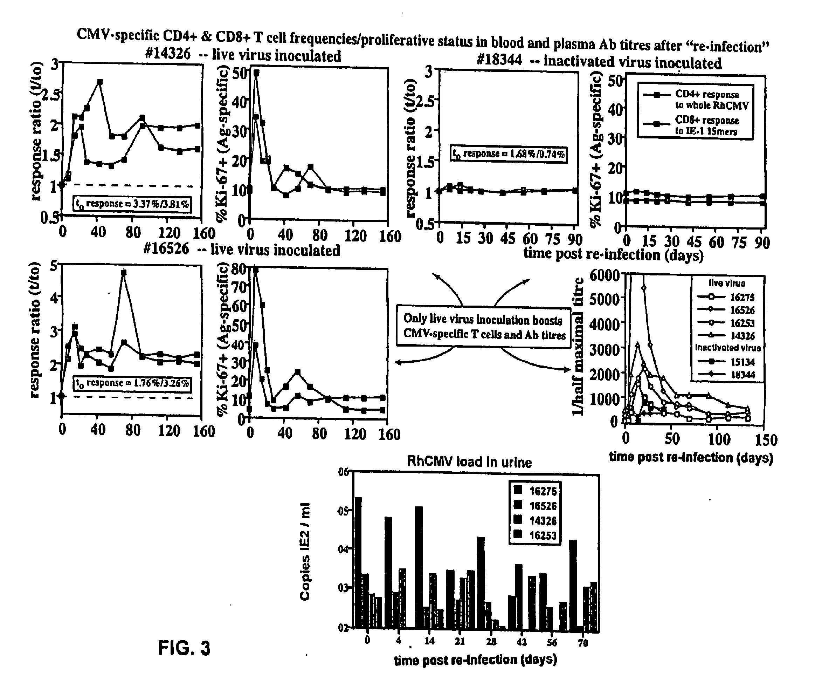

The homology between HCMV and RhCMV infections extends to their respective immune responses as well. As shown below and in FIGURE 2A and 2B, the RM T cell response to RhCMV in peripheral blood is similar to that in the human in both size and the CMV ORFs targeted. Moreover, the increased accessibility of the RM model has allowed investigation on the frequencies of these RhCMV-specific T cells in tissue sites, and the immunologic response to RhCMV re-infection. With regard to the former, as illustrated in FIGURE 2C, the representation of CMV-specific T cells in the memory repertoire at the pulmonary tissue:air interface can be truly enormous, often more than 10-fold higher than in peripheral blood. With regard to the latter, inoculation of CMV-immune RM with live (but not inactivated) wildtype RhCMV results in a dramatic boosting of CMV-specific T cell (both CD4+ and CD8+) and Ab responses (FIGURE 3). The boosted Ab titres appear to return to baseline after about 2 months, but importantly, the peripheral blood frequencies of CMV-specific CD4+ and CD8+ T cells appear to stabilize at levels 50-100% higher than their previous baseline—suggesting periodic re-infections or overt re-activations substantially contribute to the high frequency of RhCMV-specific T cells observed in most adult RM.

*CMV as a vaccine vector for HIV*/*SIV.* According to particular aspects of the present invention, many of the recognized characteristics of CMV make this virus highly attractive as a vaccine vector, particularly for SIV and HIV. *First* and foremost, CMV's capability to elicit strong Ab responses and enormous T cell responses, focused on mucosal sites (FIGURE 2) is of obvious relevance to SIV/HIV host defense (see below). Applicants emphasize that these strong responses reflect a steady state situation, maintained indefinitely, rather than the post-boost peak responses that are often highlighted in prior art vaccine studies.

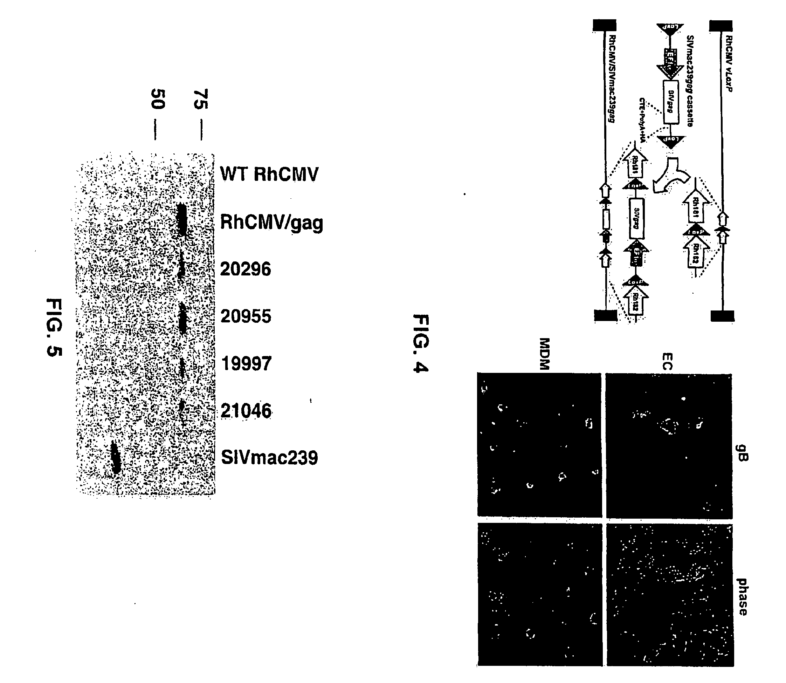

*Second,* CMV has the ability to re-infect immune hosts, and generate new immune responses with such re-infections (106, 107). To confirm this phenomenon directly with RhCMV, applicants constructed a particular RhCMV vector embodiment encoding SIV gag (FIGURE 4A). This vector showed clear-cut gag expression by both immunofluorescence (FIGURE 4B) and western blot, and demonstrated *in vitro* growth kinetics indistinguishable from that of wildtype virus. When subcutaneously administered (5 x 10⁶ pfu) to 4 RhCMV seropositive RM (224 days after primary infection), a similar (clinically aymptomatic) boosting of the RhCMV-specific T cell and Ab response as described in FIGURE 3 was observed. As applicants had previously observed in re-infection (FIGURE 3), real time PCR did not identify RhCMV, either wildtype or the gag-recombinant, in blood or lung lavage mononuclear cells at any time point following viral inoculation. However, urine from day 127 post re-infection was weakly positive for gag expression by ELISA, suggesting the presence of the RhCMV-gag vector. These samples were co-cultured to isolate RhCMV, and these *in vivo*-derived viral preparations were assessed for gag expression by western blot. As shown in FIGURE 5, RhCMV co-cultures from all 4 animals expressed immunoreactive gag, definitively establishing the presence of the administered recombinant virus in secretory sites. Retrospective analysis of urine at earlier post-re-infection time points revealed the presence of the gag-expressing RhCMV vector by day 7 in one RM and by day 21 in the other 3. Moreover, the gag-expressing RhCMV vector has also been detected in saliva, and remains present in urine at least through day 237 post re-infection.

These data have two critical implications. *First,* they unequivocally demonstrate that CMV is capable of re-infecting immune subjects, effectively competing with pre-existent wildtype virus, and somehow finding its way to its usual 'ecological' niche despite strongly boosted cellular and humoral immunity directed at CMV. *Second,* they demonstrate the *in vivo* stability of RhCMV vectors expressing exogenous neoAgs, indicating that these vectors are able to persistently infect inoculated subjects, and indefinitely maintain expression of the inserted, exogenous Ag-encoding genes.

This RhCMV-gag re-infected cohort was also used to assess the ability of RhCMV vectors to initiate a *de novo* immune response in the face of the massive CMV-specific memory boost associated with re-infection.

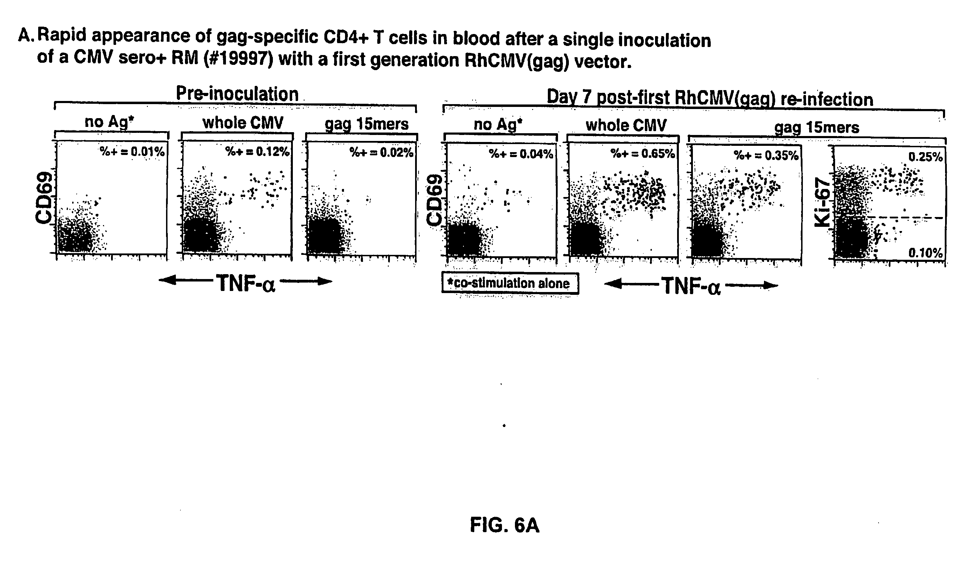

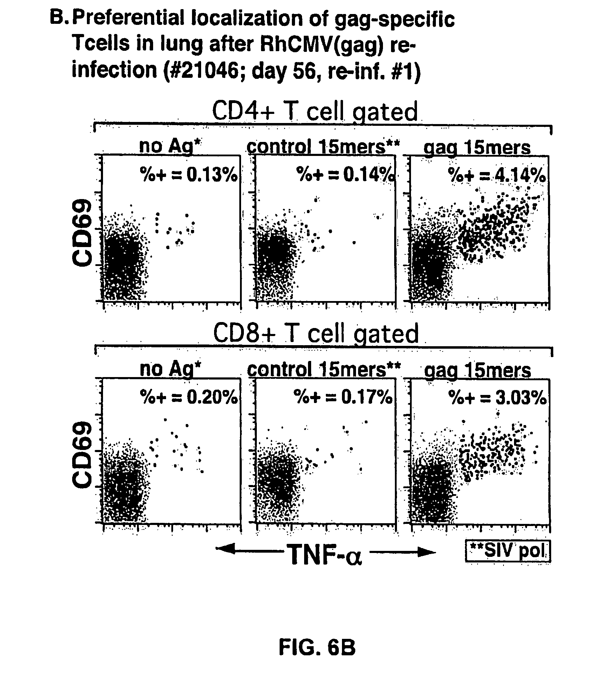

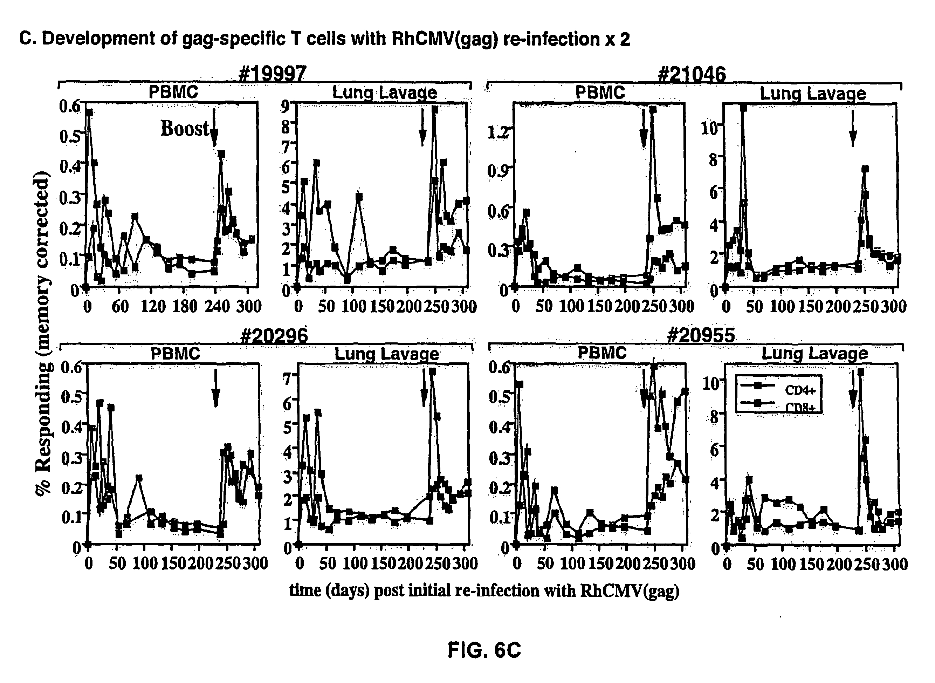

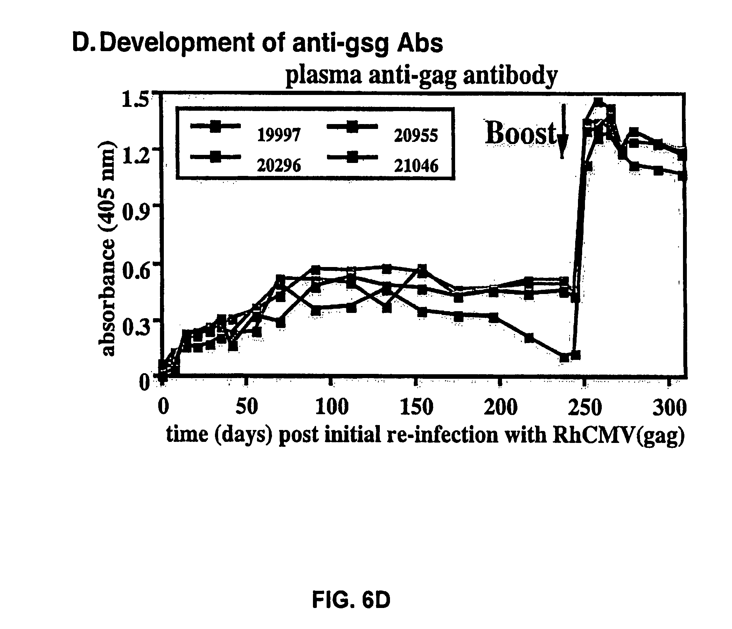

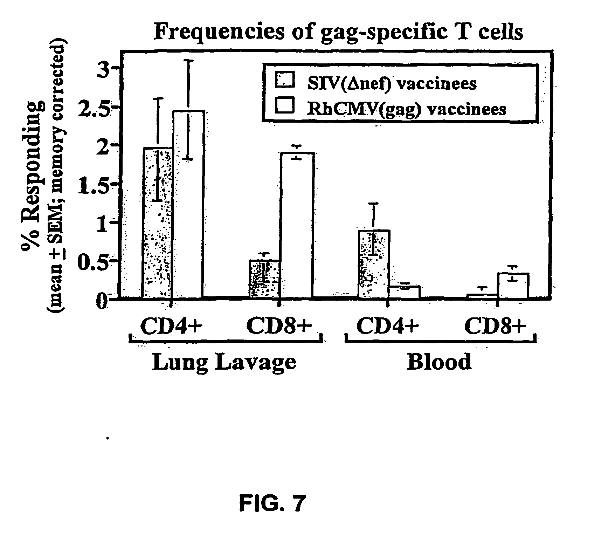

As shown in FIGURES 6A and 6C, all inoculated RM developed gag-specific CD4+ and CD8+ T cell responses in blood, as early as day 7 post re-infection. These blood responses peaked in the first month following re-infection at 0.2%-0.6% of memory cells, declining thereafter to a stable plateau in the 0.1%-0.2% range. SIV gag-specific Abs were also induced by day 14 pi, increasing through day 91 pi, prior to achieving a stable plateau (FIGURE 6D). Re-administration of the same RhCMV-gag vector (@ day 238 post re-infection) dramatically boosted both examined), these responses remained higher than the previous 'set points,' suggesting establishment of higher plateau levels. Significantly, as previously shown for RhCMV-specific T cell responses, blood frequencies of these CMV-vectored gag-specific T cell responses substantially (>10X) underestimated the frequency of SIV-specific T cells in a tissue effector site—the lung (FIGURES 6B and 6C). Gag-specific CD4+ and CD8+T cells were found in lung lavage fluid by day 7 post the initial RhCMV(gag) re-infection in all RM, peaking with frequencies as high as 10% (day 42-56 post re-infection) before achieving stable plateau levels in the 1%-3% range. Boosting (second re-infection) resulted in a sharp spike in these frequencies with return to the previous plateau level in 2 RM, and what appear to be (at day 70 post re-infection #2) slightly higher plateau levels in the other 2 animals. Significantly, the frequency and distribution of gag-specific T cells in these RhCMV(gag)-immunized RM are comparable to what applicants have observed in RM 'immunized' with attenuated SIVmac239(Δnef) that effectively controlled I.V. challenge with wildtype SIVmac239 (FIGURE 7). It should also be noted that in all of the RM studied, the observed gag-specific responses occurred in the setting of significant boosting of the RhCMV-specific responses (FIGURE 6A; indeed, it is possible these recall CMV-specific responses acted as an adjuvant for the new gag-specific response). Significantly, these data demonstrate the ability of RhCMV to function effectively as a vector for neoAgs in RhCMV-immune RM.

According to particular aspects of the present invention, the unique ability of CMV to effectively 'vector' neoAg responses in CMV-immune subjects has several highly significant implications for their utility in a HIV/SIV vaccine. *First,* by virtue of the fact that any healthy CMV+ subjects have operationally demonstrated their ability to control wildtype CMV infection, their risk of morbidity after administration of CMV vectors, even vectors based on an otherwise unmodified CMV genome, would be expected to be minimal. Although fetuses of CMV+ mothers can in some circumstances be infected (106), this risk can be averted by simply not providing CMV-based vaccines to pregnant females. Incorporation of safety (*e.g*., inducible suicide) mechanisms and/or strategic gene deletion (so as to reduce pathogenic potential without sacrificing immunogenicity and persistence) would be expected to reduce the possibility of vaccine morbidity even further. It should also be noted that other well-studied herpesviruses that could potentially be used as persistent and perhaps re-infection capable vectors have one or more features that mitigate against such use. For example, the γ-herpeviruses Epstein Barr Virus (EBV) and Kaposi's Sarcoma Herpes Virus (KSHV) are firmly associated with malignancies, whereas CMV is not. Additionally, α herpesviruses (herpes simplex) lack the generalized T cell immunogencity of CMV and because of neurovirulence (*e.g*., herpes encephalitis) would appear to have considerable more pathogenic potential in otherwise immunocompetent individuals.

The *second* implication of CMV's re-infection capability and applicants demonstration that a subsequent inoculation with the same RhCMV vector elicits a strong immunologic boost to the recombinant (SIV) gene product (FIGURE 6C) is that, unlike essentially all other viral vectors currently in use or in development, CMV vectors can likely be used repeatedly. Indeed, according to particular embodiments of the present invention, individuals are serially vaccinated with different CMV vectors so as to generate responses to new epitopes, and broadening the vaccinee's response repertoire to, for example, encompass HIV clade/strain variation.

*Significance.* As reviewed above, HIV's and SIV's remarkable ability to replicate, generate variants, and evolve past any selective pressure in days to weeks poses a substantial barrier for the immunologic control of this infection by vaccination. By all accounts, the most effective way, if not the only way, to immunologically contain HIV/SIV is to strongly attack it early, in tissue sites of first invasion, when the viral population is small viral genetic variation is at a minimum, and the host's immunologic capabilities have not been degraded. In natural infection, the immune response almost always lags behind explosive viral replication, never catches up, and is progressively degraded by viral pathogenicity.

However, according to particular aspects of the present invention, a CMV-driven, anti-HIV/SIV immune response (*e*.*g*., in place at the time of HIV/SIV exposure) is sufficient to contain viral replication, blunt the initial viral diaspora, prevent immunopathogenicity, and establish a non-progressive infection. Aspects of the present invention co-opt CMV's eons of evolution, and provide strategically engineered and optimized CMV as an HIV/SIV ORF-encoding vaccine vector. CMV's combination of 1) remarkable immunogenicity, 2) low pathogenicity, 3) persistence, 4) widespread tissue dissemination, and 5) the ability to re-infect CMV+ hosts is substantially and fundamentally different from other AIDS vaccine vectors in development, making this a truly novel approach to the AIDS vaccine problem. Indeed, the issue of persistence, by itself, makes this approach substantially worthy. According to particular aspects, long-term Ag exposure, as is possible with the inventive CMV-based vaccine vectors, correlates with a qualitatively different and functionally superior anti-lentiviral immune response.

For safety considerations, CMV vectors, with all their unique potential, must be handled appropriately. Therefore, in preferred aspects, the inventive CMV-based vaccination vectors are used to elicit neoAg immunity in CMV+ hosts, who have—by their healthy, CMV+ status— established their ability to contain this virus. Although no live virus vector is without risk, in the appropriate setting (say in the context of demonstrably immunocompetent, CMV+ preadolescents), the risk of serious CMV vector pathogencity, even with non-safety modified vectors, should be minimal. Certainly, in a population at high risk of HIV infection, any CMV vaccine-related morbidity would pale in comparison to the devastation wrought by AIDS (consider sub-Saharan Africa where early establishment of chronic CMV infection is nearly universal, and the incidence of HIV infection is up to 30%). Applicants point out that a live HCMV vaccine developed by MedImmune (formerly Aviron) is in phase 1 clinical trials at the present time. This HCMV vaccine is designed to prevent neonatal CMV infection, a serious problem, but one that is not nearly of the magnitude of the AIDS epidemic. It is important to note that the MedImmune vaccine is intended for CMV-naïve individuals, whose potential for symptomatic disease is much higher than for the CMV-infected/immune individuals targeted by applicants present vaccine embodiments. Finally, according to further aspects of the present invention, even the relatively low disease-inducing potential of wildtype CMV is abrogatable by genetic manipulation of the CMV vector without sacrificing immunogenicity or persistence.

According to aspects of the present invention, CMV vectors, alone or in combination with other modalities, provide qualitatively superior cellular and humoral immune responses against pathogenic R5-tropic SIV such that such challenges are significantly contained. Additional aspects provide safe vectors without sacrificing the unique persistence and re-infection capabilities. According to yet further aspects, various CMV genes are deletable without sacrificing vector function, and particular inventive vectors use the genomic 'space' created by such deletions for insertion of safety constructs (*e.g.*, inducible suicide mechanisms) as well as larger (poly-cistronic), SIV or HIV gene encoding cassetes. This gene deletion approach is designed to retain the balance between immunogencity/persistence and pathogencity, and exploits the redundancy of CMV's adaptations in providing engineered RhCMV optimized vectors.

In preferred aspects, RhCMV vectors are designed to reflect genes and biology that are homologous to HCMV, and optimized RhCMV vector designs are directly applicable to construction of HCMV vector embodiments.

### EXAMPLE 2

### (Preferred HCMV vectors)

Particular aspects provide recombinant RhCMV/SIV vectors, HCMV/HIV vectors, and HCMV/TB vectors that can be growth-modulated *in vivo* (*e.g.,* by oral administration of the antibiotic doxycyline). Heterologous antigen expression may be under the control of promoters of different kinetic classes with respect to the CMV infection cycle (e.g., EF1α - constitutive; MIE - immediate early; pp65 - early; gH - late).

In particular embodiments, RhCMV/SIV, HCMV/HIV and HCMV/TB vectors lack immune modulatory genes (*e*.*g*., Rh158-166 and Rh182-189) to enhance vector immunogenicity, safety and heterologous gene carrying capacity of the vector. For example, HCMV encodes at least four different gene products, gpUS2, gpUS3, gpUS6 and gpUS11 that interfere with antigen presentation by MHC I (37). All four HCMV MHC evasion molecules are encoded in the unique short region of HCMV and belong to the related *US6* gene family. Additional HCMV immunomodulators include, but are not limited to UL118, UL119, UL36, UL:37, UL111a, UL146, UL147, etc.). Likewise, RhCMV contains analogous immune modulatory genes, that can be deleted or modified to enhance vector immunogenicity, safety and heterologous gene carrying capacity of the inventive vaccine vectors.

In additional embodiments, RhCMV/SIV, HCMV/HIV and HCMV/TB are further optimized for anti-SIV/HIV/TB immunogenicity by insertion of *multiple* antigen genes (e.g., *gag, env, retanef* fusion). Alternatively, several vectors, each having a single inserted antigen may be used for co-administration.

In additional embodiments, RhCMV/SIV, HCMV/HIV and HCMV/TB vectors contain *LoxP* sites (*e.g.,* RhCMV/SIV*gagLoxPCre*) strategically placed in the CMV genome to flank an essential region of the viral genome, in combination with a tetracycline (Tet)-regulated Cre recombinase. Following immunization, doxycycline (Dox)-mediated induction of Cre recombinase enables *in vivo* inactivation of RhCMV/SIVgag*LoxPCre* by cleavage at the *LoxP* sites.

*Construction and Characterization of the RhCMV BAC.* The development of BAC technology to clone large segments of genomic DNA coupled with sophisticated λ phage-based mutagenesis systems has revolutionized the field of herpes virology enabling genetic approaches to analyze the virus. Applicants have used this system, for example, to construct an RhCMV BAC (RhCMV BAC-Cre) containing the complete RhCMV strain 68-1 genome. The RhCMV BAC-Cre was derived from an infectious, pathogenic RhCMV 68-1/EGFP recombinant virus (16). RhCMV BAC-Cre contains a BAC cassette inserted at a single *LoxP* site within the *Rh181* (*US1*)/ *Rh182* (*US2*) intergenic region of RhCMV*vLoxP*. Insertion of the BAC cassette at this site results in the generation of *LoxP* sequences flanking the cassette. As the BAC cassette contains a *Cre* gene that is expressed in eukaryotic cells, transfection of this 'self-excising' RhCMV BAC-Cre into fibroblasts results in efficient excision of the BAC cassette, reconstituting virus (designated RhCMV*vLoxP*). Characterization of the growth of the BAC-reconstituted virus (RhCMV*vLoxP*) *in vitro* and *in vivo* demonstrates that the various genetic manipulations did not alter the WT properties of the virus. The genomic structure of RhCMV*vLoxP* is identical to that of WT RhCMV except for the residual *LoxP* site. The presence of the *LoxP* sequence does not alter the expression profiles of neighboring *Rh181* (*US1*) and *Rh182* (*US2*) or distal (*IE2*) genes. RhCMV*vLoxP* replicates with WT kinetics both in tissue culture and in RhCMV seronegative immunocompetent RMs (n=2). Analysis of tissues from one animal terminated at 6 months post-inoculation demonstrated the presence of both RhCMV DNA and IE1-expressing cells in the spleen, consistent with the persistent gene expression observed in previous studies with WT virus. Both RMs developed vigorous anti-RhCMV antibody titers comparable to those observed in naturally infected animals. Taken together, these observations demonstrate that RhCMV*vLoxP* is phenotypically WT and is suitable to construct site-specific alterations for the development of vaccine vectors.

*Construction of RhCMV*/*SIKmac239gag.* The presence of the single functional *LoxP* site located in the intergenic region between *Rh181* (*US1*) and *Rh182* (*US2*) of RhCMV*vLoxP* was exploited to utilize a Cre recombinase/*LoxP* system for construction of recombinant virus. For this approach, pSIVmac239gag plasmid was used as a source of template for PCR amplification of the SIVmac239*gag* cassette. The SIVmac239*gag* cassette contains the cellular EF1α promoter driving expression of the SIVmac239*gag* gene. The EF1α promoter is a highly active promoter that is constitutively active in all cell types tested and is expected to result in high cell type independent expression of the *gag* gene. PCR amplification was performed using primers designed to incorporate a single *LoxP* site at either end of the amplified SIVmac239*gag* cassette. For recombination, the PCR product containing the SIVmac239*gag* cassette flanked by *LoxP* sites was transfected into RM fibroblasts. At 24 hours post-transfection, these cells were infected with RhCMV*vLoxP* at a multiplicity of infection (MOI) of 1. The infection was allowed to progress until extensive cytopathic effect was observed. At this time, virus-infected cells were harvested and used to infect fresh fibroblasts, which were then overlayed with agarose to prevent viral spread through the culture. After approximately two weeks, individual viral plaques were picked, and each plaque was used to infect fresh fibroblasts. Total cell lysates were then screened for the presence of the SIVmac239*gag* gene by PCR. SIVmac239*gag-*positive cell lysates were then sonicated, serial diluted and used to infect fresh fibroblasts. The process was repeated thrice, after which time, plaque-purified virus clones were screened for *gag* gene expression by northern blot analysis of total RNA obtained from infected cells (data not shown). The presence of Gag protein expression was confirmed by western analysis as well as immunofluorescence in EC and MDM). The entire SIVmac239*gag* cassette was sequenced to confirm sequence integrity of the inserted cassette. The growth kinetics of gag-positive clones were compared to WT virus and a single RhCMV/SIVmac239*gag* recombinant virus with WT growth characteristics and high levels of gag expression was selected.

At day 224 post-primary infection, RhCMV/SIVmac*239gag* was subcutaneously administered to a cohort of 4 RhCMV-seropositive RM. As we have previously observed in re-infection studies, real-time PCR did not detect RhCMV, either WT or RhCMV/SIVmac*239gag*, in blood or lung lavage mononuclear cells at any time point following viral inoculation. However, urine from day 127 post re-infection was weakly positive for gag expression by ELISA suggesting the presence of RhCMV/SIVmac*239gag*. These samples were co-cultured to isolate RhCMV, and these in vivo-derived viral preparations were assessed for gag expression by western blot. As shown in FIGURE 5, RhCMV co-cultures from all 4 animals expressed gag, definitively establishing the presence of RhCMV/SIVmac*239gag* virus at these epithelial secretory sites. Retrospective analysis of urine at earlier post-re-infection time points revealed the presence of the gag-expressing RhCMV vector in urine by day 7 in one RM and by day 21 in the other 3 (data not shown). Moreover, gag-expressing RhCMV was also present in saliva, and remained present in urine at least through day 237 post re-infection. Significantly, re-infection of this RhCMV/SIVmac239*gag* clone induced a mucosally-oriented, gag-specific CD4+ and CD8+ T cell response, as well as a gag-specific antibody response (see Figure 6B, 6C and 6D). These data unequivocally demonstrate that CMV is capable of re-infecting immune subjects, effectively competing with pre-existent WT virus, and establishing infection at normal sites within the host, despite strongly boosted cellular and humoral immunity directed at CMV. They also demonstrate the high *in vivo* stability of RhCMV vectors expressing exogenous heterologous antigens, suggesting that these vectors may be able to persistently infect inoculated subjects and indefinitely maintain expression of the inserted, exogenous antigen-encoding genes. Finally, they demonstrate that recombinant RhCMV can elicit both T cell and Ab responses to exogenous proteins in the setting of re-infection, and thus has the potential to serve as an effective vaccine vector in individuals with pre-existing CMV immunity.

### Exemplary HCMV and RhCMV vaccine vectors.

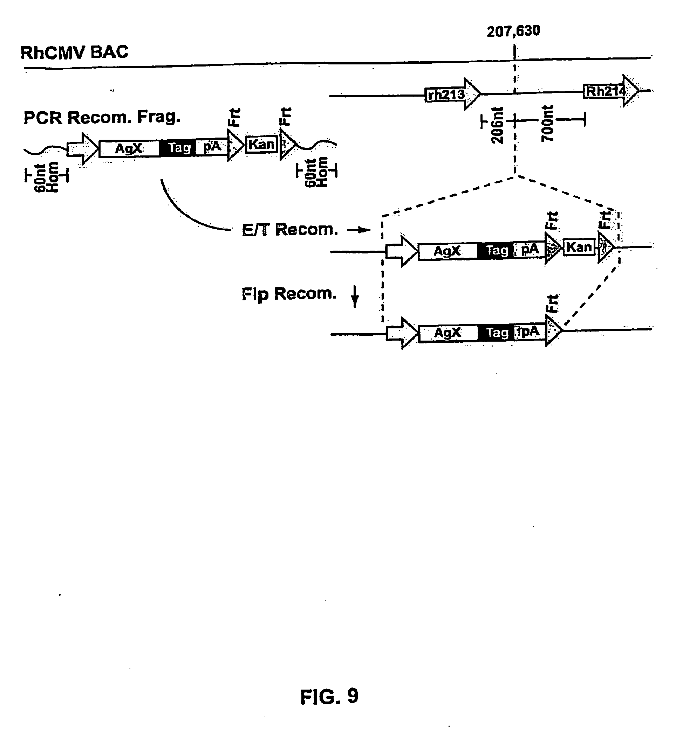

Figure 9 schematically shows construction of RhCMV and HCMV vaccine vectors according to particular aspects of the present invention. Heterologous pathogen antigen(s) are inserted into RhCMV or HCMV bacterial artificial chromosomes (BACs) by E/T and Flp-mediated recombination. The schematic shows, for example, a generalized strategy for insertion of an epitope-tagged pathogen antigen into the non-coding region between rh213 and Rh214 of RhCMV. This strategy can be similarly used for insertion of heterologous pathogen antigens at other defined sites within the RhCMV/HCMV genome, as well as insertion of multiple antigens at single or multiple sites within the genome. The gene encoding the epitope-tagged pathogen antigen is inserted into the BAC genome using E/T recombination. Following selection of recombinant BACs on the basis of antibiotic resistance (in this case, Kan), the resistance gene is removed by Flp-mediated recombination. Recombinant RhCMV and HCMV vaccine vectors are reconstituted by transfection of recombinant BACs into RhCMV/HCMV permissive cells (AgX, pathogen antigen; Tag, epitope tag; pA, polyadenylation site; Kan, kanamycin resistance gene for selection in bacteria).

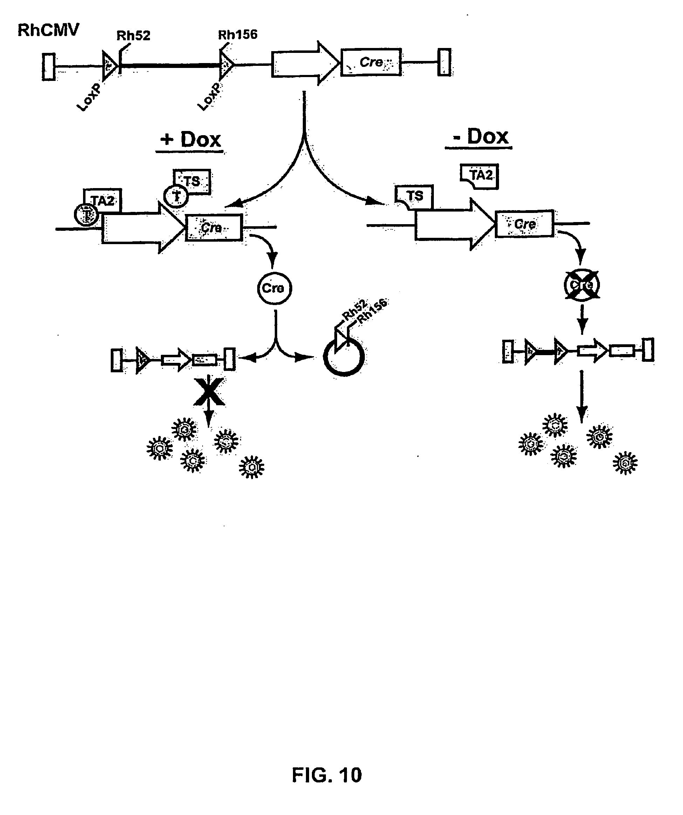

Figure 10 shows, according to particular aspects, an exemplary tetracycline-regulated RhCMV/HCMV 'safety' vaccine vector. This RhCMV/ HCMV safety vector contains two interactive genetic components within the RhCMV/HCMV genome that together enable Tet-induced vector inactivation. A Tet-inducible Cre recombinase gene (*Cre*) inserted within the viral genome enables induction of Cre recombinase expression by treatment with the Tet homologue, doxycycline (Dox) (D). This Tet-regulated system is comprised of a Tet-sensitive reverse-transactivator (rtTA2^{s}-M2) (TA2), a Tet-transrepressor (tTS-*kid*) (TS) and a tetO₇-CMV minimal promoter unit (white arrow) driving Cre-recombinase expression. The second component necessary for Tet-induced inactivation is a pair of *LoxP* sites located within the viral genome to flank a region of the genome essential for virus replication (in this case, Rh52-Rh156). In the absence of Dox, the binding of tTS-*kid* and lack of binding of rtTA2^{s}-M2 to the tetO₇-CMV minimal promoter unit prevents Cre-recombinase expression. In the absence of Cre recombinase, the integrity of the RhCMV/HCMV genome is maintained and the virus replicates normally. Addition of Dox results in the allosteric modulation of tTS-*kid* and rtTA2^{s}-M2 that results in the activation of Cre recombinase expression. Cre recombinase inactivates the RhCMV/HCMV vaccine vectors by catalysing the excision of the region of the viral genome flanked by l*oxP* sites (in this case, Rh52-Rh156). For simplicity, genes expressing rtTA2^{s}-M2 and tTS-*kid* as well as the gene expressing the heterologous pathogen antigen are not shown.

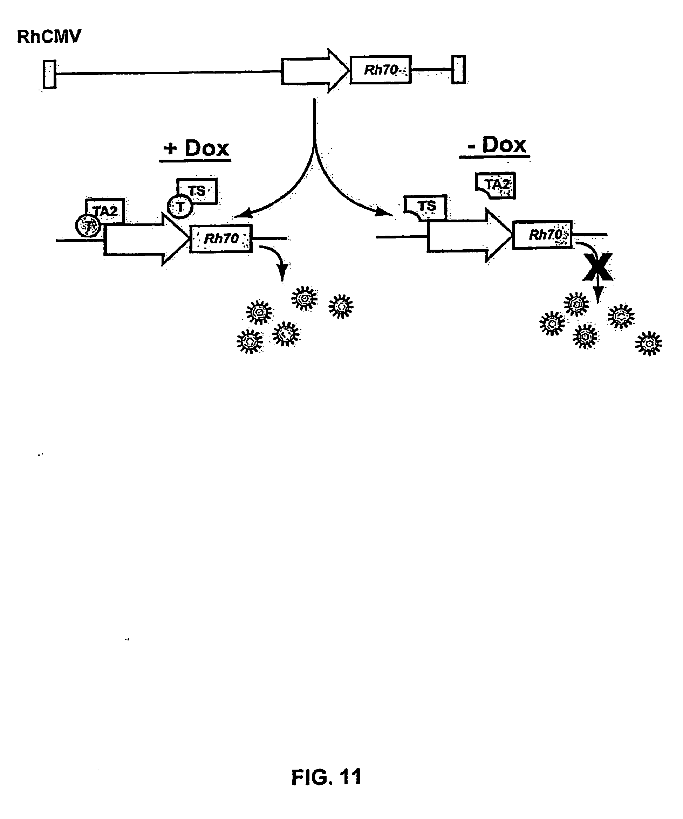

Figure 11 shows another exemplary tetracycline-regulated RhCMV/HCMV 'safety' vaccine vector. Such RhCMV/HCMV vaccine vectors are constructed by placing a gene essential for virus replication, (in this example, *Rh70* (HCMV homologue-*UL44*); DNA polymerase processivity factor), under control of the Tet-inducible system described in Figure 10. The *Rh70* HCMV homologue (*UL44*) was initially selected as this gene has been shown to be essential for CMV replication (Shenk PNAS 100:12396, 2003; Ripalti A J Virol. 1995 69:2047). However, other essential viral genes can be used as candidate genes for Tet-mediated regulation. Regulation of *Rh70* expression or expression of other essential RhCMV/HCMV genes by the Tet-regulated system enables control of virus replication by varying Dox level. To place the essential gene under Dox control, the 5' upstream region of the gene is replaced with the *tetO₇-CMV* minimal promoter unit (white arrow). After inoculation of animals with Tet-regulated vectors in the presence of Dox, virus replication can be inactivated simply by Dox withdrawal. Tet-regulated vectors are constructed by E/T and Flp-based recombination as detailed in Figure 9.

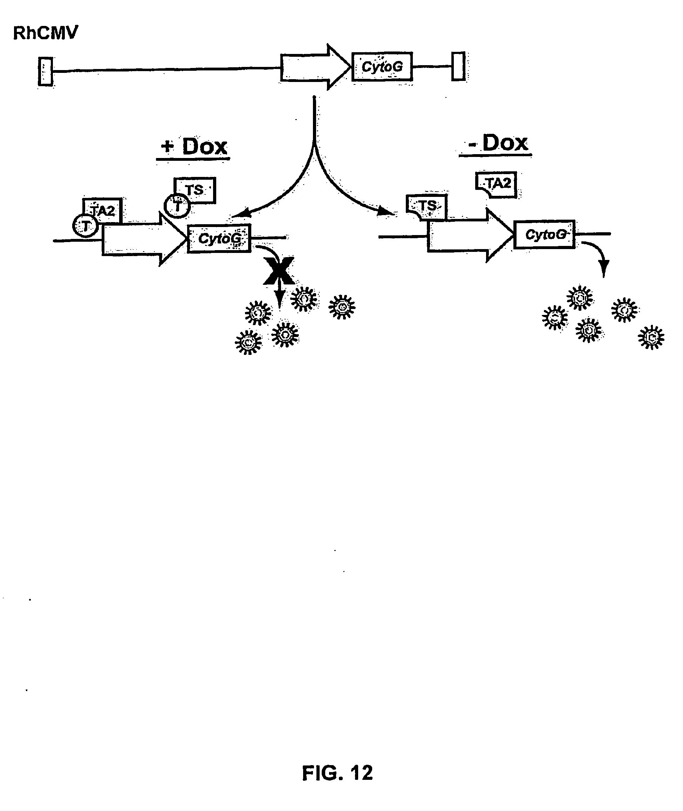

Figure 12 shows yet another exemplary tetracycline-regulated RhCMV/HCMV 'safety' vaccine vector. Such RhCMV/HCMV vaccine vectors are constructed containing a cytotoxic gene (CytoG) under control of the Tet-inducible system as detailed in Figure 11. After inoculation of animals with Tet-regulated vectors in the absence of Dox, virus replication can be rapidly inactivated by Dox-mediated induction of the cytopathic gene resulting in death of the vaccine vector-infected cell.

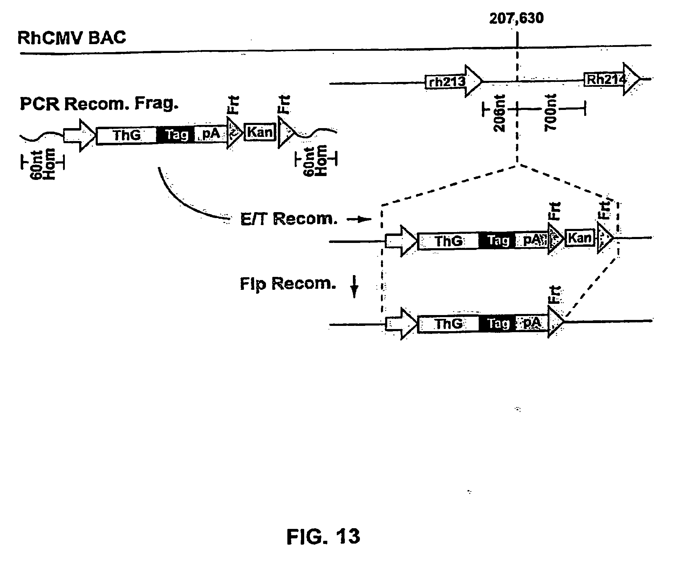

In further aspects, RhCMV and HCMV gene therapy vectors are provided. Figure 13 schematically shows construction of exemplary RhCMV and HCMV gene therapy vectors. Therapeutic gene(s) are inserted into RhCMV or HCMV bacterial artificial chromosomes (BACs) by E/T and Flp-mediated recombination. The schematic shows a generalized strategy for insertion of an epitope-tagged therapeutic gene into the non-coding region between rh213 and Rh214 of RhCMV. This strategy can be similarly used for insertion of therapeutic genes at other defined sites within the RhCMV/HCMV genome. The gene encoding the epitope-tagged replacement gene is inserted into the BAC genome using E/T recombination. Following selection of recombinant BACs on the basis of antibiotic resistance (in this case, Kan), the resistance gene is removed by Flp-mediated recombination. Recombinant RhCMV and HCMV gene therapy vectors are reconstituted by transfection of recombinant BACs into RhCMV/HCMV permissive cells (ThG, therapeutic gene; Tag, epitope tag; pA, polyadenylation site; Kan, kanamycin resistance gene for selection in bacteria).

Exemplary sequences useful in the construction and/or design of the inventive RhCMV and HCMV vaccine vectors are summarized in Table 3 below. According to aspects of the present invention, a variety to HCMV and RhCMV types and strains have utility if the inventive compositions and methods for treating and/or preventing HIV, SIV, TB, etc.

**TABLE 3. Listing of SEQ ID NOs and respective names**

| | |
|---|---|
| SEQ ID NO:1 | RhCMV (Cercopithecine herpesvirus 8) |
| SEQ ID NO:2 | HCMV (AD169 lab strain) |
| SEQ ID NO:3 | HCMV (wild type strain Merlin) |
| SEQ ID NO:4 | Towne BAC HCMV isolate |
| SEQ ID NO:5 | PH-BAC HCMV isolate |
| SEQ ID NO:6 | Toledo-BAC HCMV isolate |
| SEQ ID NO:7 | TR-BAC HCMV isolate |
| SEQ ID NO:8 | FIX-BAC HCMV isolate |
| SEQ ID NO:9 | AD 169-BAC HCMV isolate |
| SEQ ID NO:10 | SIV |
| SEQ ID NO:11 | SIV Gag-Pol |
| SEQ ID N0:12 | SIV gag protein |
| SEQ ID NO:13 | SIV vif protein |
| SEQ ID NO:14 | SIV vpx protein |
| SEQ ID NO:15 | SIV envelope protein |
| SEQ ID NO:16 | SIV nef protein |
| SEQ ID NO:17 | HIV type I |
| SEQ ID NO:18 | HIV-1 Gag-Pol |
| SEQ ID NO:19 | HIV-1 Pr55(Gag) |
| SEQ ID NO:20 | HIV-1 Vif |
| SEQ ID NO:21 | HIV-1 Vpr |
| SEQ ID NO:22 | HIV-1 Tat |
| SEQ ID NO:23 | HIV-1 Rev |
| SEQ ID NO:24 | HIV-1 Vpu |
| SEQ ID NO:25 | HIV-1 Envelope surface glycoprotein gp160, |
| SEQ ID NO:26 | HIV-1 Nef |
| SEQ ID NO:27 | HIV type 2 |
| SEQ ID NO:28 | HIV-2 gag-pol fusion polyprotein |
| SEQ ID NO:29 | HIV-2 gag polyprotein |
| SEQ ID NO:30 | HIV-2 vif protein |
| SEQ ID NO:31 | HIV-2 vpx protein |
| SEQ ID NO:32 | HIV-2 vpr protein |
| SEQ ID NO:33 | HIV-2 tat protein |
| SEQ ID NO:34 | HIV-2 rev protein |
| SEQ ID NO:35 | HIV-2 env polyprotein |
| SEQ ID NO:36 | HIV-2 nef protein |

### EXAMPLE 3

### (Treatment and/or prevention of TB)

The search for a new and improved vaccine against tuberculosis (TB) is an active field of research, which has benefited tremendously from the completed Mycobacterium tuberculosis genome and the progress in molecular biology and computer science (Andersen & Doherty, Microbes and Infection, 2005 in press).

Nonetheless, the only currently available vaccine against tuberculosis (TB), bacillus Calmette-Guerin (BCG), was derived from the virulent organism Mycobacterium bovis, a close relative of Mycobacterium tuberculosis, by growing it in vitro for 13 years until it lost the ability to cause disease in inoculated individuals. More than 3 billion people have received BCG, which makes this vaccine the most widely used in the world.

However, the safety aspects, loss of sensitivity to tuberculin as a diagnostic reagent, and varying efficacy in different trials of BCG are of great concern. While BCG likely protects children efficiently against the early manifestations of TB, estimates of protection against adult pulmonary TB have ranged from 0% to 80% based on large well-controlled field trials. This suggests that globally, BCG prevents only ∼5% of all potentially vaccine-preventable deaths due to TB, and its impact on the global TB epidemic has been negligible. A novel, effective vaccination strategy against adult pulmonary TB has therefore become an international research priority.

While most of these novel strategies are based on one or a few antigens, the BCG vaccine is a very complex vaccine that theoretically provides the immune system with more than 4000 different antigens. As a live attenuated vaccine, BCG furthermore stimulates the immune system for prolonged periods of time and it is therefore not surprising that in general these novel vaccines have had difficulty surpassing BCG's activity in animal models of TB—something that has not altered noticeably over recent decades. To include antigens that give rise to beneficial responses and exclude potential detrimental components is the ultimate advantage provided by subunit vaccines but the safety profile and possible incorporation of a TB subunit vaccine into existing pediatric vaccines also makes the subunit approach very attractive. The field is now moving towards combining important antigens to increase the coverage and efficacy of the vaccine (*Id*).

Therefore, particular aspects of the present invention provide compositions and methods for the treatment of tuberculosis (TB). TABLE 2 shows a list of representative vaccine-relevant TB antigens recognized by human T cells for use in the present inventive methods and compositions.

Particular aspects provide a method for treatment or prevention of TB, comprising infection of a subject in need thereof with at least one recombinant HCMV vector comprising an expressible TB antigen or a variant or fusion protein thereof. Preferably, the subject is an immunocompetent, TB seropositive subject. Preferably, TB antigen is selected from the group consisting of ESAT-6, Ag85A, AG85B, MPT51, MPT64, CFP10, TB10.4, Mtb8.4, hspX, CFP6, Mtb12, Mtb9.9 antigens, Mtb32A, PstS-1, PstS-2, PstS-3, MPT63, Mtb39, Mtb41, MPT83, 71-kDa, PPE 68, LppX, and antigenic portions, variants and fusion proteins thereof.

In particular embodiments, the method further comprises serial re-infection with at least one recombinant HCMV vector comprising an expressible TB antigen or a variant or fusion protein thereof. Preferably, in such embodiments, the expressible TB antigen, or variant or fusion protein thereof, of the serial re-infection vector is different than that of the initial infection vector.

In particular embodiments of the above methods, expression is driven by an antigen encoding sequence in operable association with a promoter selected from the group consisting of a constitutive CMV promoter, an immediate early CMV promoter, an early CMV promoter and a late CMV promoter. Preferably, the promoter is selected from the group consisting of EF1-alpha, MIE, pp65 and gH.

Also provided is a recombinant HCMV vaccine vector, comprising an expressible TB antigen or a variant or fusion protein thereof. Preferably, the virus comprises suicide means. In certain embodiments, expression is driven by an antigen encoding sequence in operable association with a promoter selected from the group consisting of a constitutive CMV promoter, an immediate early CMV promoter, an early CMV promoter and a late CMV promoter. Preferably, the promoter is selected from the group consisting of EF1-alpha, MIE, pp65 and gH.

Further provided are pharmaceutical compositions, comprising, along with a pharmaceutically acceptable carrier or excipient, a recombinant HCMV comprising an expressible TB antigen or a variant or fusion protein thereof. Preferably, in such compositions, the recombinant HCMV vaccine vector comprises suicide means.

**TABLE 2. Representative vaccine-relevant TB antigens recognized by human T cells for use in the present inventive methods and compositions.**

| **Antigen** | **Sanger ID** |
|---|---|
| ESAT-6 | Rv3875 |
| Ag85A | Rv3804c |
| AG85B | Rv1886c |
| MPT51 | Rv3803c |
| MPT64 | Rv1980c |
| CFP10 | Rv3874 |
| TB10.4 | Rv0288 |
| Mtb8.4 | Rv1174c |
| hspX | Rv2031c |
| CFP6 | Rv3004 |
| Mtb 12 | Rv2376c |
| Mtb9.9 antigens | Rv1793, MT3721, Rv1198, Rv1037c, Rv3619c |
| Mtb32A | Rv0125 |
| PstS-1 | Rv0934 |
| PstS-2 | Rv0932c |
| PstS-3 | Rv0928 |
| MPT63 | Rv1926c |
| Mtb39 | Rv1196 |
| Mtb41 | Rv0915c |
| MPT83 | Rv2873 |
| 71-kDa | Unknown |
| PPE68 | Rv3873 |
| LppX | Rv2945c, Rv3878, Rv3407, RV1818c |

### LITERATURE CITED

1. Grovit-Ferbas, K., T. Pappas, and W. A. O'Brien, Human Immunodeficiency Virus, in Persistent Viral Infections, R. Ahmed and A.I. Chen, Editors. 1999, John Wiley & Sons: Chicester. p. 3-45.
2. McChesney, M., E.T. Sawai, and CJ. Miller, Simian Immunodeficiency Virus, in Persistent Viral Infections, R. Ahmed and LS. Chen, Editors. 1999, John Wiley & Sons: Chichester. p. 321-345.
3. Cohen, OJ. and A.S. Fauci, Pathogenesis and Medical Aspests of HIV-I Infection, in Fields Virology, D.M. Knipe and P.M. Howley, Editors. 2001, Lippincott Williams & Wilkins: Philadelphia, p. 2043-2094.
4. Levy, J.A., Pathogenesis of human immunodeficiency virus infection. Microbiol Rev, 1993. 57(1): p. 183-289.
5. Desrosiers, R.C., Strategies used by human immunodeficiency virus that allow persistent viral replication. Nat Med, 1999. 5(7): p. 723-5.
6. Paul, W.E., Can the immune response control HIV infection? Cell, 1995. 82: p. 177-82.
7. Kaul, R., et al., New insights into HIV-I specific cytotoxic T-lymphocyte responses in exposed, persistently seronegative Kenyan sex workers. Immunol Lett, 2001. 79(1-2): p. 3- 13.
8. Rosenberg, E.S., et al., Vigorous HIV-I -specific CD4+ T cell responses associated with control ofviremia. Science, 1997. 278(5342): p. 1447-50.
9. Picker, LJ. and V.C. Maino, The CD4(+) T cell response to HW-I. Curr Opin Immunol, 2000. 12(4): p. 381-6.
10. Harrer, T., et al., Cytotoxic T lymphocytes in asymptomatic long-term nonprogressing HIV- 1 infection. Breadth and specificity of the response and relation to in vivo viral quasispecies in a person with prolonged infection and low viral load. J Immunol, 1996. 156(7): p. 2616-23.
11. Harrer, T., et al., Strong cytotoxic T cell and weak neutralizing antibody responses in a subset of persons with stable nonprogressing HTV type I infection. AIDS Res Hum Retroviruses, 1996. 12(7): p. 585-92.
12. Learmont, J.C., et al.. Immunologic and virologic status after 14 to 18 years of infection with an attenuated strain of HIV-I. A report from the Sydney Blood Bank Cohort. N Engl J Med, 1999. 340(22): p. 1715-22.
13. Shearer, G.M., HIV-induced immunopathogenesis. Immunity, 1998. 9(5): p. 587-93.
14. Hazenberg, M.D., et al., T cell depletion in HIV-I infection: how CD4+ T cells go out of stock. Nat Immunol, 2000. 1(4): p. 285-9.
15. McCune, J.M., The dynamics ofCD4+ T-cell depletion in HIV disease. Nature, 2001. 410(6831): p. 974-9.
16. Douek, D. C, LJ. Picker, and R. A. Koup, T cell dynamics in HIV-I infection. Annu Rev Immunol, 2002. 21 :265-304.
17. Schacker, T.W., et al.. Collagen deposition in HTV-I infected lymphatic tissues and T cell homeostasis. J Clin Invest, 2002. 110(8): p. 1133-1139.
18. McMichael, AJ. and S. L. Rowland- Jones, Cellular immune responses to HIV. Nature, 2001. 410(6831): p. 980-7.
19. Kostense, S., et al., Persistent numbers oftetramer(+) CD8(+) T cells, but loss of interferon-gamma(+) HIV-specific T cells during progression to AIDS. Blood, 2002. 99(7): p. 2505-11.
20. Vogel, T.U., et al., Functional impairment of simian immunodeficiency virus-specific CD8+ T cells during the chronic phase of infection. J Virol, 2001. 75(5): p. 2458-61.
21. Wyatt, R. and J. Sodroski, The HIV-I envelope glycoproteins: fusogens, antigens, and immunogens. Science, 1998. 280(5371): p. 1884-8.
22. Parren, P.W., et al.. The neutralizing antibody response to HIV-I: viral evasion and escape from humoral immunity. Aids, 1999. 13(Suppl A): p. S137-62.
23. Kimata, J.T., et al., Emerging cytopathic and antigenic simian immunodeficiency virus variants influence AIDS progression. Nat Med, 1999. 5(5): p. 535-41.
24. Schuitemaker, H., et al., Biological phenotype of human immunodeficiency virus type 1 clones at different stages of infection: progression of disease is associated with a shift from monocytotropic to T-cell-tropic virus population. J Virol, 1992. 66(3): p. 1354-60.
25. Blaak, H., et al.. In vivo HIV-I infection of CD45RA(+)CD4(+) T cells is established primarily by syncytium-inducing variants and correlates with the rate ofCD4(+) T cell decline. Proc Natl Acad Sci U S A, 2000. 97(3): p. 1269-74.
26. Scarlatti, G., et al., In vivo evolution of HTV-I co-receptor usage and sensitivity to chemokine-mediated suppression. Nat Med, 1997. 3(11): p. 1259-65.
27. Evans, D.T., et al., Virus-specific cytotoxic T-lymphocyte responses select for amino-acid variation in simian immunodeficiency virus Env and Nef. Nat Med, 1999. 5(11): p. 1270-6.
28. O'Connor, D.H., T.M. Allen, and D.I. Watkins, Cytotoxic T-lymphocyte escape monitoring in simian immunodeficiency virus vaccine challenge studies. DNA Cell Biol, 2002. 21(9): p. 659-64.
29. O'Connor, D.H., et al., Acute phase cytotoxic T lymphocyte escape is a hallmark of simian immunodeficiency virus infection. Nat Med, 2002. 8(5): p. 493-9.
30. Walker, B.D. and PJ. Goulder, AIDS. Escape from the immune system. Nature, 2000. 407(6802): p. 313-4.
31. McMichael, A., T cell responses and viral escape. Cell, 1998. 93(5): p. 673-6.
32. Finzi, D., et al., Latent infection of CD4+ T cells provides a mechanism for lifelong persistence of HTV-I, even in patients on effective combination therapy. Nat Med, 1999. 5(5): p. 512-7.
33. Saavedra Lozano, J., e al., An anti-CD45RO immunotoxin kills latently infected human immunodeficiency virus (HIV) CD4 T cells in the blood of HIV-positive persons. J Infect Dis, 2002. 185(3): p. 306-14.
34. Pitcher, CJ., et al., HIV-1-specific CD4+ T cells are detectable in most individuals with active HIV-I infection, but decline with prolonged viral suppression. Nat Med, 1999. 5(5): p. 518-25.
35. Betts, M.R., et al., Analysis of total human immunodeficiency virus (H[Iota]V)-specific CD4(+) and CD8(+) T-cell responses: relationship to viral load in untreated HIV infection. J Virol, 2001. 75(24): p. 11983-91.
36. Moore, CB., et al., Evidence of HIV-1 adaptation to HLA-restricted immune responses at a population level. Science, 2002. 296(5572): p. 1439-43.
37. Kaslow, R.A., et al., Influence of combinations of human major histocompatibility complex genes on the course of HIV-I infection. Nat Med, 1996. 2(4): p. 405-11.
38. Carrington, M., et al., HLA and HIV-I: heterozygote advantage and B*35-Cw*04 disadvantage. Science, 1999. 283(5408): p. 1748-52.
39. Schmitz, J.E., et al., Control ofviremia in simian immunodeficiency virus infection by CD8+ lymphocyte. Science, 1999. 283(5403): p. 857-60.
40. Jin, X., et al., Dramatic rise in plasma viremia after CD8(+) T cell depletion in simian immunodeficiency virus-infected macaques. J Exp Med, 1999. 189(6): p. 991-8.
41. Metzner, KJ., et al., Effects of in vivo CD8(+) T cell depletion on virus replication in rhesus macaques immunized with a live, attenuated simian immunodeficiency virus vaccine. J Exp Med, 2000.191(11): p. 1921-31.
42. Lifson. J.D., et al., Role ofCD8(+) lymphocytes in control of simian immunodeficiency virus infection and resistance to rechallenge after transient early antiretroviral treatment. J Virol, 2001. 75(21): p. 10187-99.
43. Matano, T., et al., Administration of an anti-CD8 monoclonal antibody interferes with the clearance of chimeric simian/human immunodeficiency virus during primary infections of rhesus macaques. J Virol, 1998. 72(1): p. 164-9.
44. Amara. R.R., et al., Critical role for Env as well as Gag-Pol in control of a simian-human immunodeficiency virus 89.6P challenge by a DNA prime/recombinant modified vaccinia virus Ankara vaccine. J Virol, 2002. 76(12): p. 6138-46.
45. Amara, R.R., et al., Control of a mucosal challenge and prevention of AIDS by a multiprotein DNA/MVA vaccine. Science, 2001. 292(5514): p. 69-74.
46. Amara, R.R., et al., Different patterns of immune responses but similar control of a simian- human immunodeficiency virus 89.6P mucosal challenge by modified vaccinia virus Ankara (MVA) and DNA/MVA vaccines. J Virol, 2002. 76(15): p. 7625-31.
47. Barouch, D.H., et al., Reduction of simian-human immunodeficiency virus 89.6P viremia in rhesus monkeys by recombinant modified vaccinia virus Ankara vaccination. J Virol, 2001. 75(11): p. 5151-8.
48. Barouch, D.H., et al., Control ofviremia and prevention of clinical AIDS in rhesus monkeys by cytokine-augmentcd DNA vaccination. Science, 2000. 290(5491): p. 486-92.
49. Shiver, J.W., et al., Replication-incompetent adenoviral vaccine vector elicits effective anti-immunodeficiency-virus immunity. Nature, 2002. 415(6869): p. 331-5.
50. Rose, N.F., et al., An effective AIDS vaccine based on live attenuated vesicular stomatitis virus recombinants. Cell, 2001. 106(5): p. 539-49.
51. Wyand, M.S., et al., Protection by live, attenuated simian immunodeficiency virus against heterologous challenge. J Virol, 1999. 73(10): p. 8356-63.
52. Nathanson, N., V.M. Hirsch, and BJ. Mathieson, The role of nonhuman primates in the development of an AIDS vaccine. Aids, 1999. 13(Suppl A): p. Sl 13-20.
53. HeI, Z., et al., Containment of Simian Immunodeficiency Virus Infection in Vaccinated Macaques: Correlation with the Magnitude of Virus-Specific Pre- and Postchallenge J Immunol, 2002. 169(9): p.4778-4787.
54. Pal, R., et al., ALVAC-SIV-gag-pol-env-based vaccination and macaque major histocompatibility complex class I (A*01) delay simian immunodeficiency virus SIVmac- induced immunodeficiency. J Virol, 2002. 76(1): p. 292-302.
55. Benson, J., et al., Recombinant vaccine-induced protection against the highly pathogenic simian immunodeficiency virus SIV(mac251): dependence on route of challenge exposure. J Virol, 1998. 72(5): p. 4170-82.
56. Ourmanov, L, et al., Comparative efficacy of recombinant modified vaccinia virus Ankara expressing simian immunodeficiency virus (SIV) Gag-Pol and/or Env in macaques challenged with pathogenic SIV. J Virol, 2000. 74(6): p. 2740-51.
57. Crotty, S., et al., Protection against simian immunodeficiency virus vaginal challenge by using Sabin poliovirus vectors. J Virol, 2001. 75(16): p. 7435-52.
58. Polacino, P. S., et al., Role of immune responses against the envelope and the core antigens of simian immunodeficiency virus SIVmne in protection against homologous cloned and uncloned virus challenge in Macaques. J Virol, 1999. 73(10): p. 8201-15.
59. Polacino, P., et al., Protection of macaques against intrarectal infection by a combination immunization regimen with recombinant simian immunodeficiency virus SIVmne gp160 vaccines. J Virol, 1999. 73(4): p. 3134-46.
60. Shibata, R., et al., Live, attenuated simian immunodeficiency virus vaccines elicit potent resistance against a challenge with a human immunodeficiency virus type 1 chimeric virus. J Virol, 1997. 71(11): p. 8141-8.
61. Stipp, H.L., A. Kumar, and O. Narayan, Characterization of immune escape viruses from a macaque immunized with live-virus vaccine and challenged with pathogenic SHIVKU-I. AIDS Res Hum Retroviruses, 2000. 16(15): p. 1573-80.
62. Karlsson, G.B., et al., The envelope glycoprotein ectodomains determine the efficiency of CD4+ T lymphocyte depletion in simian-human immunodeficiency virus-infected macaques. J Exp Med, 1998. 188(6): p.1159-71.
63. Parren, P.W., et al., Antibody protects macaques against vaginal challenge with a pathogenic R5 simian/human immunodeficiency virus at serum levels giving complete neutralization in vitro. J Virol, 2001. 75(17): p. 8340-7.
64. Baba, T.W., et al., Human neutralizing monoclonal antibodies of the IgGI subtype protect against mucosal simian-human immunodeficiency virus infection. Nat Med, 2000. 6(2): p. 200-6.
65. Shibata, R., et al., Neutralizing antibody directed against the HIV-I envelope glycoprotein can completely block HIV-I /SIV chimeric virus infections of macaque monkeys. Nat Med, 1999. 5(2): p. 204-10.
66. Mascola, J.R., et al., Protection of macaques against vaginal transmission of a pathogenic HIV- 1 /SIV chimeric virus by passive infusion of neutralizing antibodies. Nat Med, 2000. 6(2): p. 207-10.
67. Poignard, P., et al., Neutralizing antibodies have limited effects on the control of established HIV-I infection in vivo. Immunity, 1999. 10(4): p. 431-8.
68. Nathanson, N. and BJ. Mathieson, Biological considerations in the development of a human immunodeficiency virus vaccine. J Infect Dis, 2000. 182(2): p. 579-89.
69. Fast, P.E. and M.C. Walker, Human trials of experimental AIDS vaccines. Aids, 1993. 7(Suppl 1): p. S147-59.
70. Graham, B. S., et al., Analysis of intercurrent human immunodeficiency virus type 1 infections in phase I and II trials of candidate AIDS vaccines. AIDS Vaccine Evaluation Group, and the Correlates of HIV Immune Protection Group. J Infect Dis, 1998. 177(2): p. 310-9.
71. Connor, R.I., et al., Immunological and virological analyses of persons infected by human immunodeficiency virus type 1 while participating in trials of recombinant gp 120 subunit vaccines. J Virol, 1998. 72(2): p. 1552-76.
72. Johnson, R.P., Macaque models for AIDS vaccine development. Curr Opin Immunol, 1996. 8(4): p. 554-60.
73. Reimann, K. A., et al., A chimeric simian/human immunodeficiency virus expressing a primary patient human immunodeficiency virus type 1 isolate env causes an AIDS- like disease after in vivo passage in rhesus monkeys. J Virol, 1996. 70(10): p. 6922-8.
74. Feinberg, M.B. and J.P. Moore, AIDS vaccine models: Challenging challenge viruses. Nat Med, 2002. 8(3):p.207-10.
75. Horton, H., et al., Immunization of rhesus macaques with a DNA prime/modified vaccinia virus Ankara boost regimen induces broad simian immunodeficiency virus (SIV)-specific T-cell responses and reduces initial viral replication but does not prevent disease progression following challenge with pathogenic SIVmac239. J Virol, 2002. 76(14): p. 7187-202.
76. Barouch, D.H., et al.. Eventual AIDS vaccine failure in a rhesus monkey by viral escape from cytotoxic T lymphocytes. Nature, 2002. 415(6869): p. 335-9.
77. Gaschen, B., et al.. Diversity considerations in HIV-I vaccine selection. Science, 2002. 296(5577): p. 2354-60.
78. Daniel, M.D., et al., Protective effects of a live attenuated SIV vaccine with a deletion in the nefgene. Science, 1992. 258(5090): p. 1938-41.
79. Johnson, R.P., et al., Highly attenuated vaccine strains of simian immunodeficiency virus protect against vaginal challenge: inverse relationship of degree of protection with level of attenuation. J Virol, 1999. 73(6): p. 4952-61.
80. Titti, F., et al., Live attenuated simian immunodeficiency virus prevents super-infection by cloned SIVmac251 in cynomolgus monkeys. J Gen Virol, 1997. 78(Pt 10): p. 2529-39.
81. Khatissian, E., et al., Persistence of pathogenic challenge virus in macaques protected by simian immunodeficiency virus SIVmacDeltanef J Virol, 2001. 75(3): p. 1507-15.
82. Cranage, M.P., et al., In vivo resistance to simian immunodeficiency virus superinfection depends on attenuated virus dose. J Gen Virol, 1998. 79(Pt 8): p. 1935-44.
83. Baba, T. W., et al., Live attenuated, multiply deleted simian immunodeficiency virus causes AIDS in infant and adult macaques. Nat Med, 1999. 5(2): p. 194-203.
84. Gauduin, M.C., et al., Characterization of SIV-specific CD4+ T-helper proliferative responses in macaques immunized with live-attenuated SIV. J Med Primatol, 1999. 28(4- 5): p. 233-41.
85. Johnson, R.P., et al., Induction of vigorous cytotoxic T-lymphocyte responses by live attenuated simian immunodeficiency virus. J Virol, 1997. 71(10): p. 7711-8.
86. Corbin, A. and M. Sitbon, Protection against retroviral diseases after vaccination is conferred by interference to superinfection with attenuated murine leukemia viruses. J Virol, 1993. 67(9): p. 5146-52.
87. Stebbings, R., et al., Mechanisms of protection induced by attenuated simian immunodeficiency virus. II. Lymphocyte depletion does not abrogate protection. AIDS Res Hum Retroviruses, 1998. 14(13): p. 1187-98.
88. Stebbings, RJ., et al., Mechanisms of protection induced by attenuated simian immunodeficiency virus. Virology, 2002. 296(2): p. 338-53.
89. Nixon, D.F., et al., Simian immunodeficiency virus-specific cytotoxic T lymphocyte and protection against challenge in rhesus macaques immunized with a live attenuated simian immunodeficiency virus vaccine. Virology, 2000. 266(1): p. 203-10.
90. Mocarski, J., E.S. and CT. Courcelle, Cytomegaloviruses and their replication, in Fields Virology, D.M. Knipe and P.M. Howley, Editors. 2001, Lippincott Williams & Wilkins: Philadelphia, p. 2629-2674.
91. Pass, R.F., Cytomegalovirus, in Fields Virology, D.M. Knipe and P.M. Howley. Editors. 2001, Lippincott Williams & Wilkins: Philadelphia, p. 2675-2706.
92. Vogel, P., et al., Seroepidemologic studies of Cytomegalovirus infection in a breeding population of rhesus macaques. Lab Anim Sci, 1994. 44(1): p. 25-30.
93. Ho, M., Epidemiology of Cytomegalovirus infections. Rev Infect Dis, 1990. 12 Suppl 7: p. S701-10.
94. Zanghellini, F., et al., Asymptomatic primary cytomegalovirus infection: virologic and immunologic features. J Infect Dis, 1999. 180(3): p. 702-7.
95. Lockridge, K.M., et al., Pathogenesis of experimental rhesus cytomegalovirus infection. J Virol, 1999. 73(11): p. 9576-83.
96. Pass, R.F., et al., Excretion of cytomegalovirus in mothers: observations after delivery of congenitally infected and normal infants. J Infect Dis, 1982. 146(1): p. 1-6.
97. Huff, J., et al., Development and application of a real-time PCR assay for detection of B virus and Rhesus Cytomegalovirus in Rhesus Macaques. J. Gen. Virol., 2002. 84(in press.).
98. Soderberg-Naucler, C. and J. Y. Nelson, Human cytomegalovirus latency and reactivation - a delicate balance between the virus and its host's immune system. Intervirology, 1999. 42(5-6): p. 314-21.
99. Reinke, P., et al.. Mechanisms of human cytomegalovirus (HCMV) (re) activation and its impact on organ transplant patients. Transpl Infect Dis, 1999. 1(3): p. 157-64.
100. Mocarski, E.S., Jr., Immunomodulation by cytomegaloviruses: manipulative strategies beyond evasion. Trends Microbiol, 2002. 10(7): p. 332-9.
101. Atalay, R., et al., Identification and expression of human cytomegalovirus transcription units coding for two distinct Fcgamma receptor homologs. J Virol, 2002. 76(17): p. 8596-608.
102. Penfold, M.E., et al., Cytomegalovirus encodes a potent alpha chemokine. Proc Natl Acad Sci U S A, 1999. 96(17): p. 9839-44.
103. Skaletskaya, A., et al., A cytomegalovirus-encoded inhibitor of apoptosis that suppresses caspase-8 activation. Proc Natl Acad Sci U S A, 2001. 98(14): p. 7829-34.
104. Benedict, C.A., et al., Cutting edge: a novel viral TNF receptor superfamily member in virulent strains of human cytomegalovirus. J Immunol, 1999. 162(12): p. 6967-70.
105. Spencer, J.V., et al., Potent immunosuppressive activities of cytomegalovirus-encoded interleukin-10. J Virol, 2002. 76(3): p. 1285-92.
106. Boppana, S.B., et al., Intrauterine transmission of cytomegalovirus to infants of women with preconceptional immunity. N Engl J Med, 2001. 344(18): p. 1366-71.
107. Plotkin, S.A., et al., Protective effects of Towne cytomegalovirus vaccine against low- passage cytomegalovirus administered as a challenge. J Infect Dis, 1989. 159(5): p. 860-5.
108. Waldrop, S.L., et al., Determination of antigen-specific memory/effector CD4+ T cell frequencies by flow cytometry: evidence for a novel, antigen-specific homeostatic mechanism in HIV-associated immunodeficiency. J Clin Invest, 1997. 99(7): p. 1739-50.
109. Waldrop. S.L., et al., Normal human CD4+ memory T cells display broad heterogeneity in their activation threshold for cyokine synthesis. J Immunol, 1998. 161(10): p. 5284-95.
110. Asanuma, H., et al., Frequencies of memory T cells specific for varicella-zoster virus, herpes simplex virus, and cytomegalovirus by intracellular detection of cytokine expression. J Infect Dis, 2000. 181(3): p. 859-66.
111. Komanduri, K. V., et al., Direct measurement ofCD4+ and CD8+ T-cell responses to CMV in HIV-I- infected subjects. Virology, 2001. 279(2): p. 459-70.
112. Komanduri, K.V., et al., Restoration of cytomegalovirus-specific CD4+ T-lymphocyte responses after ganciclovir and highly active antiretroviral therapy in individuals infected with HIV-I. Nat Med, 1998. 4(8): p. 953-6.
113. Sester, M., et al., Sustained high frequencies of specific CD4 T cells restricted to a single persistent virus. J Virol, 2002. 76(8): p. 3748-55.
114. Sester, M., et al., Age-related decrease in adenovirus-specific T cell responses. J Infect Dis, 2002. 185(10): p. 1379-87.
115. Gillespie, G.M., et al., Functional heterogeneity and high frequencies of cytomegalovirus- specifc CD8(+) T lymphocytes in healthy seropositive donors. J Virol, 2000. 74(17): p. 8140-50.
116. Kern, F., et al., Target structures of the CD8(+)-T-cell response to human cytomegalovirus: the 72-kilodalton major immediate-early protein revisited. J Virol, 1999. 73(10): p. 8179-84.
117. Kern, F., et al., Cytomegalovirus (CMV) phosphoprotein 65 makes a large contribution to shaping the T cell repertoire in CMV-exposed individuals. J Infect Dis, 2002. 185(12): p. 1709-16.
118. Khan, N., et al.. Cytomegalovirus seropositivity drives the CD8 T cell repertoire toward greater clonality in healthy elderly individuals. J Immunol, 2002. 169(4): p. 1984-92.
119. Jarvis, M. A. and J. A. Nelson, Human cytomegalovirus persistence and latency in endothelial cells and macrophages. Curr Opin Microbiol, 2002. 5(4): p. 403-7.
120. Alford, C. A., K. Hayes, and W. Britt, Primary cytomegalovirus infection in pregnancy: comparison of antibody responses to virus-encoded proteins between women with and without intrauterine infection. J Infect Dis, 1988. 158(5): p. 917-24.
121. Hayes, K., C. Alford, and W. Britt, Antibody response to virus-encoded proteins after cytomegalovirus mononucleosis. J Infect Dis, 1987. 156(4): p. 615-21.
122. Zaia, J.A., et al.. Polypeptide-specific antibody response to human cytomegalovirus after infection in bone marrow transplant recipients. J Infect Dis, 1986. 153(4): p. 780-7.
123. Rasmussen, L., et al., Virus-specific IgG and IgM antibodies in normal and immunocompromised subjects infected with cytomegalovirus. J Infect Dis, 1982. 145(2): p. 191-9.
124. Britt, WJ. and CA. Alford, Cytomegalovirus, in Fields Virology, B.N. Fields, D.M. Knipe, and P.M. Howley, Editors. 1996, Raven Press: New York.
125. Letvin, N.L., Progress in the development of an HIV-I vaccine. Science, 1998. 280(5371): p. 1875-80.
126. Swanson, R., E. Bergquam, and S.W. Wong, Characterization of rhesus cytomegalovirus genes associated with anti- viral susceptibility. Virology, 1998. 240(2): p. 338-48.
127. Lockridge, K.M., et al., Primate cytomegaloviruses encode and express an IL-10-like protein. Virology, 2000. 268(2): p. 272-80.
128. Kuhn, E.M., et al., Immunohistochemical studies of productive rhesus cytomegalovirus infection in rhesus monkeys (Macaca mulatto) infected with simian immunodeficiency virus. Vet Pathol, 1999. 36(1): p. 51-6.
129. Kaur, A., et al., Decreased frequency of cytomegalovirus (CMV)-specific CD4+ T lymphocytes in simian immunodeficiency virus-infected rhesus macaques: Inverse relationship with CMVviremia. J Virol, 2002. 76(8): p. 3646-58.
130. Sequar, G., et al., Experimental coinfection of rhesus macaques with rhesus cytomegalovirus and simian immunodeficiency virus: pathogenesis. J Virol, 2002. 76(15): p. 7661-71.
131. Tarantal, A.F., et al., Neuropathogenesis induced by rhesus cytomegalovirus in fetal rhesus monkeys (Macaca mulatta). J Infect Dis, 1998. 177(2): p. 446-50.
132. Kravitz, R.H., et al.,. Cloning and characterization of rhesus cytomegalovirus glycoprotein B. J Gen Virol, 1997. 78(Pt 8): p. 2009-13.
133. Barry, P.A., et al., Nucleotide sequence and molecular analysis of the rhesus cytomegalovirus immediate-early gene and the UL1 21-117 open reading frames. Virology, 1996. 215(1): p. 61-72. 134. Connor, R.I., et al. Temporal analyses of virus replication, immune responses, and efficacy in rhesus macaque immunized with a live, attenuated simian immunodeficiency virus vaccine. J Virol, 1998. 72(9): p. 7501-9.

## Claims

1. A recombinant HCMV vaccine vector, comprising an expressible TB antigen or a variant or fusion protein thereof.

2. The recombinant vector of claim 1, comprising suicide means.

3. The recombinant vector of claim 1 or 2, wherein expression is driven by an antigen encoding sequence in operable association with a promoter selected from the group consisting of a constitutive CMV promoter, an immediate early CMV promoter, an early CMV promoter and a late CMV promoter.

4. The recombinant vector of claim 3, wherein promoter is selected from the group consisting of EF1-alpha, MIE, pp65 and gH.

5. A pharmaceutical composition, comprising, along with a pharmaceutically acceptable carrier or excipient, a recombinant vector according to any one of claims 1 to 4.

6. A recombinant HCMV vector comprising an expressible TB antigen or a variant or fusion protein thereof for use in a method for treatment or prevention of TB, comprising infection of a subject in need thereof with at least one of said vectors.

7. The vector of claim 6 for use in a method for treatment or prevention of TB according to claim 6, wherein infection is of an immunocompetent, TB seropositive subject.

8. The vector of claim 6 for use in a method for treatment or prevention of TB according to claim 6, wherein at least one TB antigen is selected from the group consisting of ESAT-6, Ag85A, AG85B, MPT51, MPT64, CFP10, TB10.4, Mtb8.4, hspX, CFP6, Mtb12, Mtb9.9, antigens, Mtb32A, PstS-1, PstS-2, PstS-3, MPT63, Mtb39, Mtb41, MPT83, 71-kDa, PPE 68, LppX, and antigenic portions, variants and fusion proteins thereof.

9. The vector of claim 6 for use in a method for treatment or prevention of TB according to claim 6, wherein the method further comprises serial re-infection with at least one of said vectors.

10. The vector for use of claim 9, wherein the expressible antigen, or variant or fusion protein thereof, of the serial re-infection vector is different than that of the initial infection vector.

11. The vector of claim 6 for use in a method for treatment or prevention of TB according to claim 6, wherein expression is driven by an antigen encoding sequence in operable association with a promoter selected from the group consisting of a constitutive CMV promoter, an immediate early CMV promoter, an early CMV promoter and a late CMV promoter.

12. The vector of claim 11, wherein the promoter is selected from the group consisting of EF1-alpha, MIE, pp65 and gH.

## Patentansprüche

1. Rekombinanter HCMV Impfstoffvektor, umfassend ein exprimierbares TB-Antigen oder eine Variante oder ein Fusionsprotein davon.

2. Rekombinanter Vektor nach Anspruch 1, umfassend Suizidmittel.

3. Rekombinanter Vektor nach Anspruch 1 oder 2, wobei die Expression von einer antigenkodierenden Sequenz in funktionsfähiger Verbindung mit einem Promotor gesteuert wird, der aus der Gruppe bestehend aus einem konstitutiven CMV-Promotor, einem unmittelbaren frühen (immediate early) CMV-Promotor, einem frühen (early) CMV-Promotor und einem späten (late) CMV-Promotor ausgewählt ist.

4. Rekombinanter Vektor nach Anspruch 3, wobei der Promotor aus der Gruppe bestehend aus EF1-alpha, MIE, pp65 und gH ausgewählt ist.

5. Pharmazeutische Zusammensetzung, umfassend zusammen mit einem pharmazeutisch annehmbaren Träger oder Hilfsstoff einen rekombinanten Vektor nach einem der Ansprüche 1 bis 4.

6. Rekombinanter HCMV-Vektor, umfassend ein exprimierbares TB-Antigen oder eine Variante oder ein Fusionsprotein davon zur Verwendung in einem Verfahren zur Behandlung oder Verhinderung von TB, umfassend die Infektion einer Zielperson, die der Behandlung bedarf, mit mindestens einem der Vektoren.

7. Vektor nach Anspruch 6, zur Verwendung in einem Verfahren zur Behandlung oder Verhinderung von TB nach Anspruch 6, wobei die Infektion bei einer immunkompetenten, TB-seropositiven Zielperson erfolgt.

8. Vektor nach Anspruch 6 zur Verwendung in einem Verfahren zur Behandlung oder Verhinderung von TB nach Anspruch 6, wobei mindestens ein TB-Antigen aus der Gruppe bestehend aus ESAT-6, Ag85A, AG85B, MPT51, MPT64, CFP10, TB10.4, Mtb8.4, hspX, CFP6, Mtb12, Mtb9.9, Antigenen, Mtb32A, PstS-1, PstS-2, PstS-3, MPT63, Mtb39, Mtb41, MTP83, 71-kDa, PPE 68, LppX und antigenen Abschnitten, Varianten und Fusionsproteinen davon ausgewählt ist.

9. Vektor nach Anspruch 6 zur Verwendung in einem Verfahren zur Behandlung oder Verhinderung von TB nach Anspruch 6, wobei das Verfahren weiter die serielle Reinfektion mit mindestens einem der Vektoren umfasst.

10. Vektor zur Verwendung nach Anspruch 9, wobei sich das exprimierbare Antigen oder eine Variante oder ein Fusionsprotein davon des Vektors der seriellen Reinfektion von dem des anfänglichen Infektionsvektors unterscheidet.

11. Vektor nach Anspruch 6 zur Verwendung in einem Verfahren zur Behandlung oder Verhinderung von TB nach Anspruch 6, wobei die Expression von einer antigenkodierenden Sequenz in funktionsfähiger Verbindung mit einem Promotor gesteuert ist, der aus der Gruppe bestehend aus einem konstitutiven CMV-Promotor, einem unmittelbaren frühen (immediate early) CMV-Promotor, einem frühen (early) CMV-Promotor und einem späten (late) CMV-Promotor ausgewählt ist.

12. Vektor nach Anspruch 11, wobei der Promotor aus der Gruppe bestehend aus EF1-alpha, MIE, pp65 und gH ausgewählt ist.

## Revendications

1. Vecteur de vaccin HCMV recombinant, comprenant un antigène de la tuberculose pouvant être exprimé ou une variante ou protéine de fusion de ce dernier.

2. Vecteur recombinant selon la revendication 1, comprenant des moyens de suicide.

3. Vecteur recombinant selon la revendication 1 ou 2, dans lequel l'expression est provoquée par une séquence codant l'antigène en association fonctionnelle avec un promoteur choisi parmi le groupe composé d'un promoteur constitutif de CMV, d'un promoteur de CMV précoce immédiat, d'un promoteur de CMV précoce et d'un promoteur de CMV tardif.

4. Vecteur recombinant selon la revendication 3, dans lequel le promoteur est choisi parmi le groupe composé d'EF1-alpha, de MIE, de pp65 et de gH.

5. Composition pharmaceutique, comprenant, ensemble avec un porteur ou excipient pharmaceutiquement acceptable, un vecteur recombinant selon l'une quelconque des revendications 1 à 4.

6. Vecteur de HCMV recombinant comprenant un antigène de la tuberculose pouvant être exprimé ou une variante ou protéine de fusion de ce dernier destiné à être utilisé dans une méthode de traitement ou de prévention de la tuberculose, comprenant l'infection d'un sujet qui en a besoin par au moins l'un desdits vecteurs

7. Vecteur selon la revendication 6 destiné à être utilisé dans une méthode de traitement ou de prévention de la tuberculose selon la revendication 6, dans lequel l'infection est d'un sujet séropositif de la tuberculose immunocompétent.

8. Vecteur selon la revendication 6, destiné à être utilisé dans une méthode de traitement ou de prévention de la tuberculose selon la revendication 6, dans lequel au moins un antigène de la tuberculose est choisi parmi le groupe composé d'ESAT-6, d'Ag85A, d'AG85B, de MPT51, de MPT64, de CFP10, de TB10.4, de Mtb8.4, de hspX, de CFP6, de Mtb12, de Mtb9.9, d'antigènes, de Mtb32A, de PstS-1, de PstS- 2, de PstS- 3, de MPT63, de Mtb39, de Mtb41, de MPT83, de 71-kDa, de PPE 68, de LppX, et de parties antigéniques, de variantes et des protéines de fusion de ces derniers.

9. Vecteur selon la revendication 6, destiné à être utilisé dans une méthode de traitement ou de prévention de la tuberculose selon la revendication 6, dans lequel la méthode comprend par ailleurs la réinfection en série par au moins l'un desdits vecteurs.

10. Vecteur destiné à être utilisé selon la revendication 9, dans lequel l'antigène pouvant être exprimé, ou la variante ou protéine de fusion de ce dernier, du vecteur de réinfection en série est différent de celui du vecteur de l'infection initiale.

11. Vecteur selon la revendication 6, destiné à être utilisé dans une méthode de traitement ou de prévention de la tuberculose selon la revendication 6, dans lequel l'expression est provoquée par une séquence codant l'antigène en association fonctionnelle avec un promoteur choisi parmi le groupe composé d'un promoteur constitutif de CMV, d'un promoteur de CMV précoce immédiat, d'un promoteur de CMV précoce et d'un promoteur de CMV tardif.

12. Vecteur selon la revendication 11, dans lequel le promoteur est choisi parmi le groupe composé d'EF1-alpha, de MIE, de pp65 et de gH.
